(19) Europäisches Patentamt
European Patent Office
Office européen des brevets

(11) Publication number: **0 066 310**
**B1**

(12) **EUROPEAN PATENT SPECIFICATION**

(45) Date of publication of patent specification: **20.03.85**

(51) Int. Cl.[4]: **B 01 D 53/34**, C 01 B 17/05, C 10 K 1/08

(21) Application number: **82200553.4**

(22) Date of filing: **06.05.82**

(54) **Sulphur recovery process.**

(30) Priority: **26.05.81 US 267201**
**26.05.81 US 267182**
**26.05.81 US 267197**
**26.05.81 US 267202**
**18.11.81 US 322486**
**24.11.81 US 324357**
**24.11.81 US 324359**
**26.03.82 US 362337**
**26.03.82 US 362338**

(43) Date of publication of application:
**08.12.82 Bulletin 82/49**

(45) Publication of the grant of the patent:
**20.03.85 Bulletin 85/12**

(84) Designated Contracting States:
**BE DE FR GB IT NL**

(56) References cited:
**EP-A-0 011 517**
**US-A-4 009 251**
**US-A-4 091 073**
**US-A-4 243 648**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Blytas, George Constantin**
**14323 Apple Tree**
**Houston Texas 77079 (US)**
Inventor: **Diaz, Zaida**
**11606 Village Place**
**Houston Texas 77077 (US)**

(74) Representative: **Aalbers, Onno et al**
**P.O. Box 302**
**NL-2501 CH The Hague (NL)**

**EP 0 066 310 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the removal of $H_2S$ from a sour gaseous stream, in which process

a) the sour gaseous stream is contacted in a contacting zone at a temperature below the melting point of sulphur with a reactant solution containing an effective amount of an oxidizing reactant comprising one or more ions of polyvalent metals and/or one or more chelate compounds of polyvalent metals, and

b) a sweet gaseous stream is separated from an admixture containing crystalline sulphur and a reduced reactant, and subsequently

c) at least a portion of the said crystalline sulphur is isolated.

The presence of significant quantities of $H_2S$ and $CO_2$ in various "sour" industrial gaseous streams poses a persistent problem. A gaseous stream is named "sour" if it contains significant quantities of $H_2S$ and/or $CO_2$.

Although various procedures have been developed to remove and recover these contaminants, most such processes are deficient, for a variety of reasons. One such process, of the type mentioned in the beginning, is described in U.S. patent specification 4,009,251. The sour gaseous stream is contacted, preferably with a solvent which contains a regenerable reactant, to produce solid elemental sulphur which is recovered either prior or subsequent to regeneration. Suitable reactants include ions of polyvalent metals, such as of iron, vanadium, copper, manganese, and nickel, and include chelates of polyvalent metals. Preferred reactants are coordination complexes in which the polyvalent metals form chelates with specified organic acids.

In yet another process, e.g. that disclosed in U.S.—A—4,091,073, $CO_2$ present in the gaseous stream is also removed by the use of a suitable absorbent selective for $CO_2$.

A problem associated with such processes is that the solid sulphur produced is of poor quality, i.e., it is very finely divided and difficult to separate from the reactant solution. A process which provides for the efficient reaction of $H_2S$ and removal of the sulphur produced could have great economic importance.

It is an object of the present invention to provide an economical and efficient method for the reaction of $H_2S$ and for the removal of the sulphur produced.

Another object is to produce sulphur crystals which are of increased size.

A further object is to address the problem of degradation or decomposition of the polyvalent metal chelate.

Accordingly, the invention provides a process for the removal of $H_2S$ from a sour gaseous stream, in which process

a) the sour gaseous stream is contacted in a contacting zone at a temperature below the melting point of sulphur with a reactant solution containing an effective amount of an oxidizing reactant comprising one or more ions of polyvalent metals and/or one or more chelate compounds of polyvalent metals, and

b) a sweet gaseous stream is separated from an admixture containing crystalline sulphur and a reduced reactant, and subsequently

c) at least a portion of the said crystalline sulphur is isolated, characterized in that an effective amount of a modifier comprising one or more alkanols having from 4 to 18 carbon atoms per molecule and/or one or more alkenols having from 4 to 22 carbon atoms per molecule is added to the reaction solution in step a) and is recovered together with the reduced reactant in the admixture of step b).

The sulphur crystals present in the admixture separated in step b), due to the presence of the modifier, are of improved quality, i.e. they have increased size. Hence, the isolation of the crystalline sulphur in step c) is easier. The manner of isolating the sulphur is a matter of choice. For example, the crystals may be isolated by settling, filtration, liquid flotation, or by suitable devices such as a hydrocyclone, etc. The sulphur crystals have, for example, improved filterability.

The modifier is supplied in the reactant solution in an effective or modifying amount, i.e., an amount sufficient to improve the quality of the sulphur produced. This amount will clearly be a minor amount, i.e., less than about 6 per cent by weight, based on the weight of the total reactant solution and normally will not be an amount which exceeds substantially the solubility of the given modifier in the reactant solution. In general, those skilled in the art may adjust the amount of modifier added to produce optimum results, good results being obtained, in the case of aqueous mixtures, when the modifier is present in an amount of from about 0.01 per cent by weight, based on the weight of the aqueous reactant solution to an amount which is at or near the saturation level of the modifier in the reactant solution without forming a significant second layer or phase. Accordingly, the amount employed will normally range (depending on the alkanol) from about 0.01 per cent to about 4 per cent by weight, based on the weight of the reactant solution, or slightly greater. The precise amount employed may be determined by experimentation, it being generally observed that the higher the molecular weight of the alkanol employed, the lower the concentration required to improve sulphur quality.

The reduced reactant may be given any suitable destination, but for economical reasons the

reactant is preferably regenerated. So, the reactant may be regenerated in a regeneration zone by contacting the reduced reactant with an oxygen-containing gas, producing a regenerated reactant-containing admixture. At least a portion of the sulphur crystals may be isolated prior or subsequent to regenerating the reactant. In other words, the reactant may be regenerated subsequent to step b) and prior to step c) or subsequent to step c).

The regenerated reactant-containing admixture may be used in any suitable manner; preferably, the process according to the invention is operated as a cyclic procedure by returning this admixture, subsequent to the isolation of sulphur, to the contacting zone of step a), the modifier still being present in the returned admixture.

The reduced polyvalent metal ions and/or reduced polyvalent metal chelate compounds are regenerated by contacting them with an oxygen-containing gas. Examples of oxygen-containing gases are air, air enriched with oxygen and pure oxygen. The oxygen oxidizes the reduced metal ions or the metal of the chelate or chelates to a higher valence state, and the regenerated mixture is suitably returned to the contact zone of step a), suitably after sulphur removal.

The reactant solution in step a) is suitably an aqueous solution in those cases where $H_2S$ is the only contaminant to be removed from a sour gaseous stream.

As stated hereinbefore, the sulphur crystals can easily be separated. In this connection, it has surprisingly been found that the presence of the said modifier in aqueous solution during the regeneration of the reactant can give rise to the formation of a froth from which the sulphur can easily be recovered. As a result of this finding a preferred embodiment of the prsent invention comprises producing the regenerated reactant-containing aqueous admixture and recovering the crystalline sulphur by using the following substeps:—

d) separating a froth containing sulphur and aqueous admixture from the regeneration zone,

e) contacting the froth separated in substep d) with a first liquid extracting composition comprising one or more alkanols having from 4 to 15 carbon atoms per molecule and/or one or more alkenols having from 4 to 20 carbon atoms per molecule in an amount sufficient to extract more than 50 per cent of the sulphur from the froth and forming an upper liquid phase comprising solid sulphur and said first extracting composition in contact with a lower phase comprising regenerated aqueous admixture;

f) separating at least a portion of the upper liquid phase, and

g) recovering sulphur from the portion separated in substep f).

The above sulphur removal using said froth is efficient and yields good quality sulphur. Because the first extracting composition contacts only a minor portion of aqueous admixture and sulphur in the froth, the separation is efficient, and equipment of reduced size may be employed. Should minor quantities of sulphur remain in the lower phase, the lower phase (or a portion thereof) may be contacted with an additional amount of the said first extracting composition to provide further sulphur removal and at least a portion of the additional first extracting composition containing sulphur may be separated. In either case, the regenerated reactant admixture from the lower phase in substep e) may, if desired, be returned to the contacting zone. The first extracting composition is generally present in an amount at or near the saturation level thereof in the reactant solution. Concomitantly, the bulk of the regenerated aqueous admixture from the regeneration is preferably returned to the contacting zone and is mixed there with the regenerated reactant admixture from the lower phase in substep e). In this manner the first extracting composition from substep e) becomes modifier in step a). If necessary or desirable, additional means, such as filters, may be provided during the return of the regenerated aqueous admixture for removal of any sulphur remaining in the admixture.

The upper phase separated in substep f), after sulphur recovery in substep g), comprises the first extracting composition and is therefore preferably returned to substep e) for further contacting with froth. If, as indicated hereinbefore, the regenerated reactant solution derived from the froth is subjected to an additional extraction, the first extracting composition employed, being lightly loaded, may be sent, if desired, for contact with the froth, rather than being subjected to immediate sulphur recovery.

Preferably, the formation of the said froth is carried out in a suitable device which provides intimate contacting of the oxygen-containing gas and the aqueous admixture, the oxygen-containing gas being supplied in sufficient quantity and velocity to accomplish both regeneration and "frothing" out or flotation of the sulphur. Because the volume of sulphur, admixture, etc. will vary depending on the circumstances, precise quantities and velocities of the oxygen-containing gas cannot be given. In general, however, the volume of oxygen-containing gas supplied will be at least that amount necessary to regenerate the polyvalent metal compound or chelate, or mixtures thereof, and preferably will be supplied in a stoichiometric excess of 50 to 100 percent. Formation of the sulphur-solution froth, per se, is accomplished in the regeneration zone according to well-known principles, and in well-known devices, as illustrated; for example, in Chemical Engineers Handbook, Third Edition, (1950) by Perry, pages 1085 through 1091, and Encyclopedia of Chemical Technology, 1966, by Kirk-Othmer, Vol. 9, pages 380 to 398, and references cited in each. For example, the contactor may comprise a vertical column with the oxygen-containing gas and sulphur-containing aqueous admixture entering in the lower part of the column, froth being removed near the top of the column and spent oxygen-containing gas being removed overhead. Gases other than oxygen may be present in significant quantity, such as

$CO_2$, and may be added to assist in the flotation, so long as they do not interfere with the regeneration or separation. Again, it is not intended that substantial quantities of solvents other than water be employed. Because the amount of the first extracting composition required is dependent on the amount of sulphur produced, which is, in turn, dependent on the content of $H_2S$ in the gaseous stream to be treated, precise amounts of the first extracting composition cannot be given. Those skilled in the art may adjust the amount, as required.

In general, the amount will range from 50 per cent to 300 percent by volume, based on the polyvalent metal ion solution and/or polyvalent metal chelate solution in the froth, with an amount of 100 percent to about 200 per cent by volume being preferred.

The solid sulphur apparently is suspended preferentially in the first liquid extracting composition and may be recovered easily. The manner of recovering the sulphur after suspension is a matter of choice. For example, after separating the suspension (or a portion thereof) the sulphur may be recovered by settling, filtration, or by suitable devices such as a hydrocyclone. Preferably, however, the sulphur is melted, allowing separation by the simple expedient of allowing the sulphur to settle.

It has furthermore surprisingly been found that the alkanols which are present in the modifier described hereinbefore are able to extract sulphur from aqueous admixture containing crystalline sulphur and reactant or reduced reactant. As a result of this finding a preferred embodiment of the present invention comprises recovering at least the bulk of the crystalline sulphur by using the following substeps:—

k) contacting aqueous admixture separated in step b) or regenerated reactant-containing aqueous admixture with a second liquid extracting composition comprising one or more alkanols having from 4 to 15 carbon atoms per molecule,

l) forming an upper liquid phase comprising more than 50 per cent of the solid sulphur and said second extracting composition in contact with a lower phase comprising aqueous admixture containing reduced reactant or regenerated reactant, respectively,

m) separating at least a portion of the upper phase, and

n) recovering sulphur from the portion separated in substep m).

Aqueous admixture is suitably separated from the lower phase in substep l), and, if this admixture contains reduced reactant, contacted with an oxygen-containing gas, producing a regenerated reactant-containing aqueous admixture. The second extracting composition is generally present in an amount at or near the saturation level thereof in this aqueous admixture. By returning regenerated reactant-containing aqueous admixture to step a) the second extracting composition from substep k), present in the regenerated reactant-containing aqueous admixture, becomes modifier in step a).

Suitably, at least a portion of the upper phase separated in substep m) is, after sulphur removal in substep n), returned to substep k), and contacted with aqueous admixture separated in step b) or with regenerated reactant-containing aqueous admixture. The upper phase is suitably removed continuously from the lower phase. The second extraction composition is supplied in an amount sufficient to remove at least the bulk of the sulphur from the admixture and forms a separate phase comprising sulphur and the second extracting composition. Because the amount of the second extracting composition required is dependent on the amount of sulphur produced, which is, in turn, dependent on the content of $H_2S$ in the gaseous stream to be treated, precise amounts of the second extracting composition cannot be given. Those skilled in the art may adjust the amount, as required. In general, the amount will range from 1.0 per cent to 200 per cent by volume, calculated on the aqueous polyvalent metal ion or polyvalent metal chelate solution, with an amount of 5.0 per cent to 120 per cent by volume being preferred. The solid sulphur apparently is suspended preferentially in the second extracting composition, and may be recovered easily. The manner of recovering the sulphur is a matter of choice. For example, after separating the suspension (or a portion thereof), the sulphur may be recovered by settling, filtration, or by suitable devices such as a hydrocyclone. Preferably, however, the sulphur is melted, allowing separation by the simple expedient of allowing the sulphur to settle.

It has been found that the stability of some reactants employed is temperature-dependent, i.e., if the temperature of the solutions is too high, some of the reactants tend to degrade or decompose. In particular, if temperatures above the melting point of sulphur are employed, some systems, such as particular iron chelate systems, tend to decompose.

On the other hand, if a solvent is employed to extract the sulphur from the solution, problems may arise if the solvent exhibits significant solubility in the gaseous stream treated. Thus, a need has existed for a gas treating system which would avoid the problems mentioned. This need is satisfied by separating the aqueous admixture separated in step b) or regenerated reactant-containing aqueous admixture into an aqueous solution containing reduced reactant or regenerated reactant, respectively, and having reduced sulphur content and an aqueous reactant slurry having increased sulphur content and contacting the said aqueous reactant slurry having increased sulphur content in substep k) with the said second extracting composition. Accordingly, the invention substantially reduces the possibility of reactant degradation by extracting the sulphur from a limited volume of admixture. Concomitantly, in the case of first separating sulphur and then regenerating the reduced reactant, the lower phase, or a portion thereof, may then be separated, again preferably continuously, and the reactant therein regenerated by contacting the admixture in a regeneration zone or zones with an oxygen-containing

gas. The reactant admixture may be regenerated separately, or, preferably, is regenerated in the same regeneration zone as the main stream of admixture. In the case of first regenerating the reduced reactant and then separating sulphur, the lower phase is preferably returned to the contacting zone in step a).

A critical feature of this embodiment of the invention is the separation of the sulphur-containing admixture from the contacting zone or from the regeneration zone into two portions, a portion or stream having reduced sulphur content, and a portion or stream containing increased sulphur content, preferably a slurry. The manner of separation is a matter of choice, and equipment such as a hydrocyclone or a centrifugal separator may be employed. It is not necessary that absolutely all sulphur be removed on a continuous basis in the process; the process may suitably be operated with a very minor inventory or significantly reduced content of sulphur in the system. In general, whether the sulphur is separated prior to or after regeneration is a matter of choice, the important aspect being the contacting of the limited volume of the sulphur-containing admixture or "slurry" with the second extracting composition. Preferably, the "slurry" or concentrated stream will comprise 2 per cent to 30 per cent, by volume (on a continuous basis) of the total of the aqueous admixture separated in step b) or of the regenerated reactant-containing aqueous admixture.

The process according to the present invention is very suitable for the removal of $H_2S$ and $CO_2$ from sour gaseous streams. So, in another embodiment of the invention, a sour gaseous stream containing $H_2S$ and $CO_2$ is contacted in step a) with a reactant solution also containing a liquid absorbent solution for $CO_2$, the reactant solution and procedure in steps a), b) and c) being similar to that described hereinbefore. The absorbent is preferably selective for $H_2S$ as well. A purified or "sweet" gaseous stream is produced which meets general industrial and commercial $H_2S$ and $CO_2$ specifications. The $CO_2$ is absorbed and the $H_2S$ is immediately converted to sulphur by the polyvalent metal ion and/or polyvalent metal chelate.

In the process, the reactant is reduced, and the sulphur may be treated, as described hereinbefore. The sulphur crystals may be removed prior or subsequent to a regeneration of the admixture; the crystals produced are of increased size. Preferably, if the volume of $CO_2$ absorbed is large, the reactant-containing solution is treated, such as by heating or pressure reduction, to remove the bulk of the $CO_2$ before regeneration of the reactant (either before or after sulphur removal). Alternatively, or if small quantities of $CO_2$ are absorbed, the $CO_2$ may simply be stripped in the regeneration zone.

As indicated hereinbefore, the invention also provides in this embodiment for the regeneration of the reactant and the absorbent. Specifically, the loaded absorbent admixture and the reduced polyvalent metal ions, polyvalent metal chelate, or mixtures thereof, are regenerated by contacting the mixture in a regeneration zone or zones with an oxygen-containing gas. The oxygen accomplishes two functions, the stripping of any $CO_2$ from the loaded absorbent admixture, and the oxidation of the reduced reactant to a higher oxidation state. The oxygen (in whatever form supplied) is supplied in a stoichiometric equivalent or excess with respect to the amount of reactant present in the mixture. Preferably, the oxygen is supplied in an amount in the range of from 20 per cent to 200 percent stoichiometric excess.

The alkanols used in the present process have the general formula $C_nH_{2n+1}OH$. The letter n is an integer in the range of from 4 to 18, preferably 4 to 12, for the alkanols in step a) and of from 4 to 15 (preferably 10 through 14) for the alkanols in the first and second extracting composition. The alkenols have the general formula $C_nH_{2n-1}OH$, in which n is an integer in the range of from 4 to 22 for the alkenols in step a) and from 4 to 20 (preferably 14 through 18) for the alkenols in the first extracting composition. As those skilled in the art will recognize, several of the compounds or compositions are solids at ordinary temperatures, and heat, as necessary, will be provided to convert the solid to liquid for the extraction (and maintain the compound or composition as a liquid, if necessary). In general, compounds or compositions according to the invention which have a melting point of 60°C or below (preferably 45°C, or below) are preferred as first and second liquid extracting composition. Particularly preferred alkanols in step a) are t-butanol, n-pentanol, n-octanol, n-decanol, n-undecanol, n-dodecanol, and mixtures thereof. These alkanols may also be used in the first and second liquid extracting composition. Alkenols which may be used in step a) and in the first extracting composition are 9-octa-decen-1-ol (also referred to as "oleyl alcohol"), 3-buten-1-ol, 4-methyl-1-hepten-4-ol, and mixtures thereof.

The modifier in step a) is suitably the same as the first and second extracting composition in step f) and k), respectively.

The particular type of gaseous stream treated is not critical, as will be evident to those skilled in the art. Streams particularly suited to removal of $H_2S$ and $CO_2$ by the process of the invention are, as indicated, naturally occurring gases, synthesis gases, process gases, and fuel gases produced by gasification procedures, e.g., gases produced by the gasification of coal, petroleum, shale, tar sands, etc. Particularly preferred are coal gasification streams, natural gas streams and refinery feedstocks composed of gaseous hydrocarbon streams, especially those feedstocks having a low ratio of $H_2S$ to $CO_2$, and other gaseous hydrocarbon streams. The words "hydrocarbon stream(s)", as employed herein, are intended to include streams containing significant quantities of hydrocarbon (both paraffinic and aromatic), it being recognized that such streams may contain significant "impurities" not technically

defined as a hydrocarbon. Streams containing principally a single hydrocarbon, e.g., ethane, are eminently suited to the process of the invention. Streams derived from the gasification and/or partial oxidation of gaseous or liquid hydrocarbon may be treated according to the invention. The $H_2S$ content of the type of gaseous streams contemplated will vary extensively, but, in general, will range from 0.1 per cent to 10 per cent by volume. $CO_2$ content may also vary, and may range from 0.5 per cent to over 99 per cent by volume. Obviously, the contents of $H_2S$ and $CO_2$ present are not generally a limiting factor in the process of the invention.

The temperatures employed in the contacting or absorption-contact zone in step a) are not generally critical, except that the reaction is carried out at a temperature below the melting point of sulphur. In many commercial applications, such as the removal of $H_2S$ (and, if desired, $CO_2$) from natural gas to meet pipeline specifications, contacting at ambient temperatures is desired, since the cost of refrigeration would exceed the benefits obtained due to increased absorption at the lower temperature. In general, temperatures in the range of from 10°C to 80°C are suitable, and temperatures in the range of from 20°C to 45°C are preferred. Contact times may be in the range of from 1 s to 270 s or longer, with contact times in the range of from 2 s to 120 s being preferred. Temperatures employed in the extraction of step k) will approximate those in the contacting of step a), except that they will always be below the melting point of sulphur.

Similarly, in the regeneration or in the stripping zone or zones, temperatures may be varied widely. Preferably, the regeneration zone should be maintained at substantially the same temperature as the absorption zone in step a). If heat is added to assist regeneration, cooling of the absorbent mixture is required before return of the absorbent mixture to the absorption zone. In general, temperatures in the range of from 10°C to 80°C, preferably 20°C to 45°C may be employed.

Pressure conditions in the absorption zone of step a) may vary widely, depending on the pressure of the gaseous stream to be treated. For example, pressures in the absorption zone may vary from 1 bar up to 152 or even 203 bar. Pressures in the range of from 1 bar to 101 bar are preferred. In the regeneration or desorption zone or zones, pressures may be varied considerably, and will preferably be in the range of from 0.5 bar to 3 or 4 bar. The pressure-temperature relationships involved are well understood by those skilled in the art, and need not be detailed herein. Other conditions of operation for this type of reaction process, e.g., pH, etc., are further described in U.S.—A—3,068,065, GB—A—999,799 and U.S.—A—4,009,251. Preferably, if the iron chelate of nitrilotriacetic acid is used as a reactant, pH in the process of the invention will range from 6 to 7.5, and the molar ratio of the nitrilotriacetic acid to the iron is from 1.2 to 1.4. The process is preferably carried out continuously.

As indicated, the $H_2S$, when contacted, is quickly converted by the oxidizing polyvalent metal ions, polyvalent metal chelate, etc. to elemental sulphur. Since many polyvalent metal compounds and polyvalent metal chelates have limited solubility in many solvents or absorbents, the polyvalent metal compounds or chelates are preferably supplied in admixture with the liquid absorbent and water. The amount of polyvalent metal compound, polyvalent metal chelate, or mixtures thereof, supplied is an effective amount, i.e., an amount sufficient to convert all or substantially all of the $H_2S$ in the gaseous stream, and will generally be on the order of at least about 1 mol per mol of $H_2S$. Ratios in the range of from about 1 or 2 mol to about 15 mol of polyvalent metal compound or chelate per mol of $H_2S$ may be used, with ratios in the range of from about 2 mol per mol to about 5 mol of polyvalent metal compound or chelate per mol of $H_2S$ being preferred. The manner of preparing the aqueous solution or admixture containing an absorbent is a matter of choice. For example, the compound or chelate may be added to the absorbent, and, if necessary, then water added. The amount of water added will normally be just that amount necessary to achieve solution of the polyvalent metal compound or chelate, and can be determined by routine experimentation. Since the polyvalent metal compound or chelate may have a significant solubility in the solvent, and since water is produced by the reaction of the $H_2S$ and the ions or chelate, precise amounts of water to be added cannot be given. In the case of absorbents having a low solubility for the polyvalent metal ion or chelate, approximately 5 per cent to 10 per cent water by volume, based on the total volume of the absorbent mixture, will generally provide solvency. Preferably, however, the polyvalents ions or chelate are added as an aqueous solution to the liquid absorbent. Where they are supplied as an aqueous solution, the amount of solution supplied may be 20 per cent to 80 percent by volume of the total absorbent admixture supplied to the absorption of step a). A polyvalent metal chelate solution will generally be supplied as an aqueous solution having a concentration in the range of from 0.1 molar to 2 or 3 molar, and a concentration of about 1.0 molar is preferred. If iron is used, the ligand to iron molar ratio may range from 1.1 to 1.6, preferably 1.2 to 1.4. In case the process according to the invention is carried out in the absence of an absorbent, the polyvalent metal ion or polyvalent metal chelate solution will generally be supplied as an aqueous solution having a concentration of from 0.1 molar to 2 molar, and a concentration of about 0.6 to 0.8 molar is preferred.

Any polyvalent metal can be used, but iron, copper and manganese are preferred, particularly iron. The polyvalent metal should be capable of oxidizing hydrogen sulphide, while being reduced itself from a higher to a lower valence state, and should then be oxidizable by oxygen from the lower valence state to the higher valence state in a typical redox reaction. Other polyvalent metals which can be used include lead, mercury, palladium, platinum, tungsten, nickel, chromium, cobalt, vanadium, titanium,

tantalum, zirconium, molybdenum and tin. The metals are normally supplied as a salt, oxide, hydroxide, etc.

Preferred reactants are coordination complexes in which polyvalent metals form chelates with an acid having the general formula:

$$(X)_{3-n}—N—(Y)_n$$

wherein n is an integer from 1 to 3, Y represents a carboxymethyl or 2-carboxyethyl group and X a 2-hydroxyethyl or 2-hydroxypropyl group or an alkyl group having from 1 to 4 carbon atoms, or

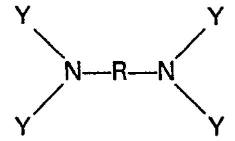

wherein:
—from 2 to 4 of the groups Y represent carboxymethyl or 2-carboxyethyl groups;
—from 0 to 2 of the groups Y represent a 2-hydroxyethyl or 2-hydroxypropyl group and
—R represents an ethylene, a trimethylene, 1-methylethylene, 1,2-cyclohexylene or 1,2-benzylene group;
or with a mixture of such acids.

The polyvalent metal chelates are readily formed in aqueous solution by reaction of an appropriate salt, oxide or hydroxide of the polyvalent metal and the chelating agent in the acid form or an alkali metal or ammonium salt thereof. Exemplary chelating agents include aminoacetic acids derived from ammonia or 2-hydroxyalkylamines, such as glycine (aminoacetic acid), diglycine (iminodiacetic acid), NTA (nitrilotriacetic acid), a 2-hydroxyalkyl glycine; a dihydroxyalkyl glycine, and hydroxyethyl- or hydroxypropyldiglycine; aminoacetic acids derived from ethylenediamine, diethylenetriamine, 1,2-propylenediamine, and 1,3-propylenediamine, such as EDTA (ethylenediaminetetraacetic acid), HEDTA (2-hydroxyethylethylenediaminetriacetic acid), DETPA (diethylenetriaminepentaacetic acid); aminoacetic acid derivatives of cyclic 1,2-diamines, such as 1,2-diaminocyclohexane-N,N-tetraacetic acid, and 1,2-phenylenediamine-N,N-tetraacetic acid, and the amides of polyaminoacetic acids disclosed in U.S.—A—3,580,950. The iron chelates of NTA and 2-hydroxyethylethylenediaminetriacetic acid are preferred.

As processes for the removal of $H_2S$ from sour gaseous streams generally represent significant costs to manufacturing operations, any improvements in such processes which increase their efficiency may have great economic importance. For example, where ligands or chelates of polyvalent metals are employed, degradation or decomposition of the polyvalent metal chelates represents an important cost in the process, as well as requiring measures for decomposition product bleed or removal and addition of fresh solution. Even in the case of preferred chelates such as those of N-(2-hydroxyethyl)ethylenediaminetriacetic acid and nitrilotriacetic acid, ligand decomposition, over a period of time, requires attention to prevent build-up of decomposition products and consequent loss of efficiency.

According to a further aspect of the invention this problem is addressed when polyvalent metal chelate-containing solutions also contain a stabilizing amount of a first stabilizing composition comprising thiodiglycolic acid and/or 3,3-thiodipropionic acid.

According to another aspect of the invention the problem of degradation or decomposition of polyvalent metal chelate-containing reactant solutions is addressed when these solutions also contain a stabilizing amount of a second stabilizing composition comprising sodium thiocyanate and/or sodium dithionite.

Thiodiglycolic acid, 3,3-thiodipropionic acid, sodium thiocyanate and/or sodium dithionate may be used in reducing the rate of degradation of the chelates employed. The first and second stabilizing compositions are supplied in a stabilizing amount, i.e., an amount sufficient to reduce or inhibit the rate of degradation. The term "stabilizing amount" thus also includes those minor amounts of the stabilizers employed, which, while perhaps not exhibiting an especially discernible reduction of ligand rate of reduction, are effective, nonetheless, in conjunction with amounts of other non-interfering and nonreactive ligand stabilizing compositions, such as those described in U.S.—A—4400368 application in reducing the ligand rate of degradation or decomposition. This amount may be determined by experimentation. In general, the amount employed of the first stabilizing composition will range from 0.005 to 0.3 mol per litre of solution, with an amount of 0.03 to 0.2 mol per litre being preferred. In general, the amount employed of the second stabilizing composition will range from 0.01 to 0.5 mol per litre of solution, with an amount of 0.05 to about 0.3 mol per litre being preferred. Those skilled in the art may adjust the amount of the first and of the second stabilizing composition added to produce optimum results, a primary consideration being simply the cost of the stabilizer added.

The absorbents employed in this invention are those absorbents which have a high degree of selectivity in absorbing $CO_2$ (and preferably $H_2S$ as well) from the gaseous streams. Any of the known

absorbents conventionally used which do not affect the activity of the polyvalent metal ions, polyvalent metal chelate, or mixtures thereof, and which exhibit sufficient solubility for the reactant or reactants may be employed. As indicated, the absorbent preferably has good absorbency for $H_2S$ as well, in order to assist in the removal of any $H_2S$ present in the gaseous mixtures. The particular absorbent chosen is a matter of choice, given these qualifications, and selection can be made by routine experimentation. For example, 3,6-dioxaoctanol (also referred to as "Carbitol" or "diethylene glycol monoethyl ether"), propylene carbonate, 2,5,8,11,14-pentaoxapentadecane (also referred to as "tetraethylene glycol dimethyl ether"), N-methylpyrrolidone, tetrahydrothiophene 1,1-dioxide (also referred to as "Sulfolane"), methylisobutyl ketone, 2,4-pentanedione, 2,5-hexanedione, 2-hydroxy-2-methyl-4-pentanone (also referred to as "diacetone alcohol"), hexyl acetate, cyclohexanone, 4-methyl-3-penten-2-one (also referred to as "mesityl oxide") and 4-methyl-4-methoxy-pentanone-2 may be used. Suitable temperature and pressure relationships for different $CO_2$-selective absorbents are known, or can be calculated by those skilled in the art.

An absorption column might comprise two separate columns in which the solution from the lower portion of the first column would be introduced into the upper portion of the second column, the gaseous material from the upper portion of the first column being fed into the lower portion of the second column. Parallel operation of units is, of course, well within the scope of the invention.

As will be undestood the solutions or mixtures employed may contain other materials or additives for given purposes. For example, U.S.—A—3,933,993 discloses the use of buffering agents, such as phosphate and carbonate buffers. Similarly, U.S.—A—4,009,251 describes various additives, such as sodium oxalate, sodium formate, sodium thiosulphate, and sodium acetate, which may be beneficial.

The invention is further illustrated by means of the following Examples.

Comparative Experiment A

$H_2S$ enters a contact vessel into which also enters an aqueous mixture containing 8.8 per cent by weight (based on the total weight of the mixture) of the Fe(III) chelate of nitrilotriacetic acid. The pressure of the feed gas is about atmospheric, and the temperature of the mixture is about 35°C. A contact time of about 100 s is employed. In the mixture, the $H_2S$ is converted to elemental sulphur by the Fe(III) chelate, Fe(III) chelate in the process being converted to the Fe(II) chelate. The sulphur produced is very fine and difficult to separate from the solution.

Example 1

A procedure similar to Comparative Experiment A is followed, except that 0.05 per cent by weight (based on the total weight of the mixture) of n-decanol is added to the reactant solution. The sulphur crystals are larger than those of Comparative Experiment A, the average particle size being approximately an order of magnitude larger in diameter.

Example 2

Sour gas, e.g., natural gas containing about 10 per cent $H_2S$ and 25 per cent by volume $CO_2$, enters an absorption vessel which contains an absorbent mixture composed of about 81 per cent sulfolane by weight (based on the total weight of the mixture), about 17 per cent by weight of an aqueous 0.5M solution of the Fe(III) chelate of 2-hydroxyethylethylenediaminetriacetic acid and about 1.7%w n-dodecanol. The pressure of the feed gas is about 3 bar, and the temperature of the absorbent mixture is about 35°C. A contact time of about 180 s is employed in order to absorb virtually all $CO_2$ and react all the $H_2S$. Purified or "sweet" gas is removed, the "sweet" gas being of a purity sufficient to meet standard requirements. In the absorbent mixture, the $H_2S$ is converted to elemental sulphur by the Fe(III) chelate, Fe(III) chelate in the process being converted to the Fe(II) chelate. The absorbent mixture, containing the elemental sulphur, absorbed $CO_2$ and the Fe(II) chelate, is removed continuously and may be stripped to regenerate the chelate and recover $CO_2$.

Examples 3—12 and Comparative Experiments B—D

A series of runs were made at a temperature of 60°C in step a) and using a procedure similar to Comparative Experiment A to determine the effect of alkanols coming within the scope of the invention. The results are presented in Table I.

TABLE I

| Example[f] or Comparative experiment[g] | Solution composition | | | Alkanol | S° mean vol. diameter (μm)[a] |
|---|---|---|---|---|---|
| | %w Fe | %m excess HOEEDTA[e] | pH | | |
| B | 8 | 10 | 4.5 | *none*[b] | 1.9 |
| 3 | 8 | 10 | 4.5 | Dodecanol (500 ppm) | 6.9 |
| 4 | 8 | 10 | 4.5 | Decanol (500 ppm) | 17.1 |
| C | 8 | 10 | 4.5 | none[d] | 5.2 |
| 5 | 8 | 10 | 4.5 | Decanol (500 ppm) | 13.7 |
| 6 | 8 | 10 | 4.5 | Decanol (5%w) | 23.8 |
| 7 | 8 | 10 | 4.5 | Neodol 91 (500 ppm)[c] | 12.3 |
| 8 | 8 | 10 | 4.5 | Neodol 91 (5%w) | 20.3 |
| D | 4 | 20 | 8 | none | 4.4 |
| 9 | 4 | 20 | 8 | Decanol (30 ppm) | 4.8 |
| 10 | 4 | 20 | 8 | Decanol (300 ppm) | 8.8 |
| 11 | 4 | 20 | 8 | Neodol 91 (30 ppm) | 4.9 |
| 12 | 4 | 20 | 8 | Neodol 91 (300 ppm) | 8.9 |

[a] Determined by Coulter Counter. This method of analysis is described in "Particle Size Measurement" by T. Allen, third edition, Powder Technology Series, edited by Chapman and Hall (1981).
[b] and [d] two different batches of the same solution recipe.
[c] "Neodol 91" is a trade mark for a mixture of primary n-alkanols having 9 to 11 carbon atoms per molecule.
[e] 2-hydroxyethylethylenediaminetriacetic acid.
[f] In Arabic numerals.
[g] In capital letters.

Example 13

Sour gas, e.g., natural gas containing about 8.0 per cent $H_2S$ and 15 per cent by volume $CO_2$, enters an absorption vessel which contains an absorbent mixture composed of 81 per cent carbitol (3,6-dioxaoctanol or diethylene glycol monoethyl ether) by weight (based on the total weight of the mixture), 17 per cent of an aqueous 0.8M solution of the Fe(III) chelate of nitrilotriacetic acid and 2.0%w dodecanol. The pressure of the feed gas is about 6.2 bar, and the temperature of the absorbent mixture is about 35°C. A contact time of about 180 s is employed in order to absorb virtually all $CO_2$ and react all the $H_2S$. Purified or "sweet" gas is removed, the "sweet" gas being of a purity sufficient to meet standard requirements. In the absorbent mixture, the $H_2S$ is converted to elemental sulphur by the Fe(III) chelate, Fe(III) chelate in the process being converted to the Fe(II) chelate. The absorbent mixture containing the elemental sulphur, absorbed $CO_2$ and the Fe(II) chelate, is removed continuously and may be stripped to regenerate the chelate and recover $CO_2$.

Comparative Experiment E

$H_2S$ enters a contact vessel into which also enters an aqueous mixture containing 1.5 per cent by weight Fe (based on the total mixture) as the Fe(III) chelate of nitrilotriacetic acid (NTA). The ligand was supplied in 40 per cent molar excess, based on iron, and the pH of the system was 7.

The pressure of the feed gas is about 1 bar, and the temperature of the mixture is about 35°C. A contact time of about 120 s is employed. In the mixture, the $H_2S$ is converted to elemental sulphur by the Fe(III) chelate, Fe(III) chelate in the process being converted to the Fe(II) chelate. The sulphur produced is very fine and difficult to separate from the solution, and has a mean volume diameter (μm) by Coulter Counter of 5.8.

Example 14

A procedure similar to Comparative Experiment E was followed, except that 300 parts per million by weight (based on the total mixture) of oleyl alcohol are added to the reactant solution. The sulphur crystals are larger than those of Comparative Experiment E, the mean volume diameter (μm) being 7.1.

Examples 15 and 16

A procedure similar to Comparative Experiment E was followed (1.5%w Fe, 40% mol excess NTA, pH=7) at a temperature of 60°C, using two different concentrations of 3-buten-1-ol. The results are presented in Table II.

TABLE II

| Example | Content of 3-buten-1-ol, %wt. | S° mean vol. diameter (μm)[b] |
|---|---|---|
| 15 | 1.5[a] | 6.4 |
| 16 | 7.5[a] | 10.3 |

[a] %wt calculated basis solution without alkenol.
[b] determined by Coulter Counter.

Example 17

This Example is described with reference to the accompanying Figure 1. As shown in this figure, a sour gaseous stream, e.g., natural gas containing about 0.5 per cent $H_2S$, in a line 1 enters a contactor or column 2 (tray type) into which also enters an aqueous admixture comprising an aqueous 2.0 M solution of the Fe(III) chelate of nitrilotriacetic acid. The pressure of the feed gas is about 84 bar, and the temperature of the aqueous admixture is about 45°C. A contact time of about 120 s is employed in order to react all the $H_2S$. Purified or "sweet" gaseous stream leaves column 2 through a line 3. The "sweet" gaseous stream is of a purity sufficient to meet standard requirements. In the admixture, the $H_2S$ is converted to elemental sulphur by the Fe(III) chelate, the Fe(III) chelate in the process being converted to the Fe(II) chelate. The aqueous admixture, containing the elemental sulphur, is removed continuously and sent through a line 4 to a depressurization and degassing unit 5, and then further via the line 4 to a sulphur extraction zone. The released gases are withdrawn from the unit 5 via a line 16. Prior to entry into a unit 6, a stream of second liquid extracting composition, in this case n-decanol, in a line 7 joins the line 4 in such a fashion that good mixing of the aqueous admixture and the decanol occurs. The decanol may, of course, be added in the unit 6, either wholly or in part, and the volume ratio of aqueous admixture to the decanol is approximately 1:1. The unit 6 is a separator in which separation takes place into an upper phase containing solid sulphur and decanol and a lower phase comprising aqueous admixture.

In the unit 6, the decanol and aqueous admixture are allowed to separate into an upper decanol layer or phase, and a lower aqueous admixture layer. Surprisingly, even though sulphur normally has a density greater than 1.0, the sulphur may be said to "float" in the liquid decanol, and is easily separated from the aqueous admixture. Large depths of a sulphur-rich zone in alkanol can be built without sulphur sinking through the aqueous-alkanol phase interface. This considerably facilitates design of the process equipment.

Decanol-sulphur mixture is removed from the separator 6 via a line 8 to a recovery zone or tank 9, where the sulphur may be removed by warming the mixture to the melting point of sulphur. Optionally, only a portion of the upper phase may be removed, a "clarified" portion being separable and recyclable, so that only a portion of the upper phase need be heated. In any event, upon melting, as shown, the sulphur sinks to the bottom of the tank 9, where it is easily removed via a line 15. Decanol is removed via a line 7, preferably after cooling, for re-use. Make-up decanol is supplied to the line 7 via a line 17.

Concomitantly, the aqueous admixture is removed via a line 10 for regeneration of the Fe(II) chelate. The decanol is present in the aqueous admixture in an amount at or near the saturation level thereof. In a regeneration column 11 the admixture is contacted with excess air in a line 12 to convert the Fe(II) chelate to the Fe(III) chelate. The temperature of the regeneration column 11 is about 45°C, and pressure in the column is maintained at about 2 bar. Spent air is removed from the column 11 through a line 13, while regenerated aqueous admixture is returned via a line 14 to the contactor 2. The decanol is present in the regenerated aqueous admixture in an amount at or near the saturation level thereof.

The drawing illustrates the aspect of the invention wherein the extraction is carried out before regeneration. Removal of the sulphur after regeneration may be preferred in some instances, and may be accomplished by positioning of the extraction unit "after" the regeneration column 11. Thus, regenerated liquid, still containing sulphur, may be passed via line 14 to units analogous or equivalent

to units 6, 7, 8, 9, 15 and 17, sulphur recovered, and regenerated sulphur-free solution returned via a line analogous to the line 10 to the contactor 2.

Example 18

This Example is described with reference to the accompanying Figure 2. Figure 2 illustrates an embodiment of the invention, wherein sulphur is removed prior to regeneration. Reference numerals in Figures 1 and 2 which are the same refer to analogous or equivalent units.

In Fig. 2, a sour gaseous mixture, e.g. natural gas containing about 0.5 per cent $H_2S$, in a line 1 enters a contactor or column 2 (tray type) into which also enters an aqueous admixture comprising an aqueous 2.0 M solution of the Fe(III) chelate of nitrilotriacetic acid from a line 14. The pressure of the feed gas is about 84 bar, and the temperature of the aqueous admixture is about 45°C. A contact time of about 120 s is employed in order to react all the $H_2S$. Purified or "sweet" gaseous stream leaves the column 2 through a line 3. The "sweet" gaseous stream is of a purity sufficient to meet standard requirements. In the admixture, the $H_2S$ is converted to elemental sulphur by the Fe(III) chelate, the Fe(III) chelate in the process being converted to the Fe(II) chelate. The aqueous admixture, containing the elemental sulphur, is removed continuously and sent through a line 4 to a depressurization and degassing unit 5, and then further via the line 4 to a separation zone 20. The released gases are withdrawn from the unit 5 via a line 16. The separation zone 20 preferably comprises a unit, such as a hydrocyclone, for separating the admixture into two portions, the major portion or stream having a reduced sulphur content (shown leaving in a line 21), and a portion or stream having an increased sulphur content (shown leaving in a line 22). It is not necessary that all sulphur be removed from the portion separated in the line 21, and some sulphur retention may be beneficial. Preferably, the amount of sulphur removed in the hydrocyclone is simply balanced with the rate of sulphur intake in the reactor 2, which is, of course, dependent on the $H_2S$ content of the gaseous stream supplied via the line 1. Those skilled in the art may appropriately adjust the rates of withdrawal of streams 21 and 22. Typically, stream 22 comprises 10 per cent by volume of the total volume of admixture in line 4.

Accordingly, the fluid slurry in the line 22 proceeds to a separation zone 6. Prior to entry into the zone 6, a stream of n-dodecanol in a line 7 joins the line 22 in such a fashion that good mixing of the aqueous admixture and the n-dodecanol occurs. The n-dodecanol may, of course, be added in the zone 6, either wholly or in part, and the volume ratio of aqueous admixture to the n-dodecanol is approximately 1:1.

In the separation zone 6, the n-dodecanol and aqueous admixture are allowed to separate into an upper n-dodecanol layer or phase, and a lower aqueous admixture layer. Surprisingly, even though sulphur normally has a density greater than 1.0, the sulphur may be said to "float" in the n-decanol, and is easily separated from the aqueous admixture. Large depths of a sulphur-rich zone in alkanol can be built without sulphur sinking through the aqueous alkanol phase interface. This considerably facilitates design of the process equipment. The n-dodecanol-sulphur mixture is removed from the separator 6 via a line 8 to a recovery zone or tank 9, where the sulphur may be removed by warming the mixture to the melting point of sulphur. Optionally, only a portion of the upper phase may be removed, a "clarified" portion being separable and recyclable, so that only a portion of the upper phase need be heated. In any event, upon melting, as shown, the sulphur sinks to the bottom of tank 9 from whence it is easily removed via a line 15. The n-dodecanol is removed via the line 7, and is re-used. Make-up dodecanol is supplied to the line 7 via a line 17. Excess heat in the solvent may be removed, preferably in unit 6 or 12, as desired. The lower aqueous layer in unit 6 is removed via a line 23; the decanol is present in the aqueous layer in an amount at or near the saturation level thereof.

Concomitantly, as noted, the aqueous admixture is removed from the separation zone 20 via the line 21 for regeneration of the Fe(II) chelate. As shown, the lower aqueous layer in the line 23 joins the line 21 prior to entry into a regeneration zone 11 although separate entry into the zone 11 is entirely feasible. In the regeneration zone or column 11 the admixture is contacted with excess air in line 12 to convert the Fe(II) chelate to the Fe(III) chelate. The temperature of the regeneration column is about 45°C, and pressure in the column is maintained at about 2 bar. Spent air is removed from the column 11 through a line 13, while regenerated aqueous admixture is returned via the line 14 to the contactor 2. Make-up aqueous reactant solution may be supplied via a line 18.

Example 19

This Example is described with reference to the accompanying Figure 3.

As indicated, Figure 2 illustrates the aspect of the invention wherein the extraction is carried out prior to regeneration. Removal of the sulphur after regeneration may be preferred in some instances, and may be accomplished by positioning of the extraction unit "after" the regeneration zone; this embodiment is illustrated in Figure 3. Reference numerals in Figures 1, 2 and 3 which are the same refer to analogous or equivalent units.

Accordingly, in Figure 3, the sulphur-containing liquid is passed, after degassing in the degassing unit 5, further via the line 4 to the regenerator 11 where it is regenerated, as previously described. The regenerated sulphur-containing admixture is removed via the line 30, and passed to the centrifugal separator 20 where it is separated into a regenerated reactant solution having reduced sulphur content

and a sulphur-containing slurry. The regenerated reactant solution is returned via the line 14 to the contactor 2, while the slurry is removed, via the line 22 to the separation zone 6. In the separation zone 6, the slurry is contacted, with n-decanol, as described in Example 18, e.g., via line 7, and regenerated reactant solution is returned, via the lines 22 and 14, to the contactor 2. Sulphur-containing decanol is removed via line 8, and may be treated to recover sulphur and decanol, in unit 9, as described in relation to Figure 2.

Example 20

This Example is described with respect to the accompanying Figures 4 and 5.

Figure 4 illustrates one embodiment of the invention, while Figure 5 illustrates another embodiment. All values are merely exemplary or calculated, and similar number designations represent similar features.

In Figure 4, a gaseous mixture, such as a natural gas containing 1 per cent $H_2S$ and 1.5 per cent $CO_2$ by volume, enters via a line 1 into a contactor or absorption column 2. Absorption column 2 is a tray contactor, although any suitable contacting device (such as a venturi) may be employed. An oxidizing reactant mixture, e.g., an aqueous mixture containing 0.4 molar Fe(III) as the complex of nitrilotriacetic acid, enters the absorption column 2 via a line 3. For illustrative purposes, it will be assumed that the gaseous mixture enters at 5,660 $Nm^3$ per hour, while the reactant mixture enters at 19 $m^3$ per hour. Pressure of the gaseous mixture in the line 1 is 70 bar, and the temperature of the gaseous mixture is 30°C. Reactant mixture is supplied at a temperature of 30°C and contact time is 45 s. The flow of liquid and gaseous mixture, as illustrated, provides for good contact and reaction of the $H_2S$ in the gaseous mixture to sulphur. As will be understood by those skilled in the art, water and the Fe(II) complex or chelate of nitrilotriacetic acid are also produced by the reaction. Sweet gaseous mixture is removed overhead via a line 4.

Reactant admixture is removed from the absorption column 2 via a line 5. From the line 5, the admixture enters a regenerator 6 which comprises a vertical contactor column. Air from a line 7 at a flow rate of 226 $Nm^3$ per hour enters the column 6 through spargers near the bottom of the column. A froth slurry is produced which is removed via a line 8. Spent air is removed overhead via a line 9, and regenerated admixture, i.e., admixture in which the bulk of the Fe(II) chelate has been oxidized to the Fe(III) chelate, is returned to the absorption column 2 via a line 10 and the line 3. Auxiliary means, such as a filter, are shown in dotted lines in line 10 to remove any residual sulphur not floated in the regenerator 6, if necessary.

The froth, which is a slurry-like mixture of sulphur and regenerated admixture, is introduced via the line 8 into a separator 11. Preferably, prior to entry into the separator 11, a stream of liquid first extracting composition, in this case n-decanol, in a line 15 joins the line 8 at a mixing device 12 in such a fashion that good mixing of the froth and the decanol occurs. In-line mixing may be used, but any suitable mixing device may be provided, if desired. For example, a Keenex mixer, or mixing devices provided with impellers may be used. Make-up decanol is supplied, if desired, via a line 19. The decanol may, of course, be added in the separator 11, either wholly or in part, and the volume ratio of aqueous admixture to the decanol is about 1:1. The combination of the decanol and intimate mixing breaks the froth.

In the separator 11, the mixture is allowed to separate into an upper decanol layer or phase, and a lower aqueous admixture layer. Surprisingly, even though sulphur normally has a density greater than 1.0, the sulphur may be said to "float" in the decanol, and is easily separated from the aqueous admixture. Large depths of a sulphur-rich zone in alkanol can be built without sulphur sinking through the aqueous-alkanol phase interface. This considerably facilitates design of the process equipment. Decanol-sulphur mixture is removed from the separator 11 via a line 13 to a recovery zone or tank 14, where the sulphur is removed, preferably by warming the mixture to the melting point of sulphur. Liquid sulphur is removed from the tank 14 via a line 18. Optionally, only a portion of the upper phase may be removed, a "clarified" portion being separable so that only a portion of the upper phase need be heated. In any event, upon melting, the sulphur sinks to the bottom of the tank 14, where it is easily removed. Decanol is removed via the line 15 for reuse, after cooling (not shown).

Concomitantly, the regenerated admixture removed from the froth is removed from the lower layer in the separator 11 via a line 16 and returned to the contactor 2, as shown. The decanol is present in the regenerated admixture in an amount at or near the saturation level thereof.

In the embodiment shown in Figure 5, sour gaseous mixture, e.g., natural gas containing about 0.5 per cent $H_2S$ and 32 per cent by volume $CO_2$, in the line 1 enters the absorption column 2 (tray type) into which also enters, via the line 3, an aqueous 0.8 M solution of the Fe(III) chelate of nitrilotriacetic acid. The pressure of the feed gas is about 83.8 bar, and the temperature of the absorbent mixture is about 45°C. A contact time of about 45 s is employed. Purified or "sweet" gaseous mixture leaves the absorption column 2 through the line 4. The "sweet" gaseous mixture is of a purity sufficient to meet standard requirements. In the absorbent mixture, the $H_2S$ is converted to elemental sulphur by the Fe(III) chelate, the Fe(III) chelate in the process being converted to the Fe(II) chelate. The mixture, containing the elemental sulphur and the Fe(II) chelate, is removed continuously

and sent through the line 5 after degassing in a unit 30, to the regeneration zone 6. Gases are withdrawn from the unit 30 via a line 31.

In the regenerator 6 the loaded mixture is contacted with excess air in the line 7, as described previously. The temperature of the regeneration column 6 is about 45°C, and pressure in the column is maintained at about 2 bar. The operation of units 6 through 12 and 18 and 19 is as described previously. However, a separator 31 receives the liquid in the line 16, and is contacted with cooled decanol from a line 32. The bulk of any sulphur remaining in the admixture is removed from the admixture and may be recovered, via a line 33, suitably in the separator 14 (or, being lightly loaded, may be used in the mixing device 12). The now fully regenerated admixture is returned via a line 17 to the absorption column 2.

Comparative Experiment F

An aqueous solution (150 ml) of the $Fe^{+++}$ chelate of nitrilotriacetic acid was placed in a vessel, and a stream of pure $H_2S$ was sparged into the solution with rapid stirring. Temperature of the solution was 35°C, and the solution contained 0.27 mol per litre iron as $Fe^{+++}$. Nitrilotriacetic acid ligand was present in 40 per cent mol excess, basis the iron. Sixty microlitres of 1-decanol (300 parts by million by weight) were also added to the solution. The pH of the solution was 7, and pressure was atmospheric. Addition of the $H_2S$ (360 ml) was continued until approximately 70 per cent of the $Fe^{+++}$ was converted to the $Fe^{++}$ state, which took about 2 to 3 min. The flow of the $H_2S$ and the stirring action were then discontinued, oxygen in excess was sparged into the solution, and stirring resumed for 15 min, thus regenerating the $Fe^{+++}$, and completing one cycle. The procedure was repeated for 5 cycles, the time of regeneration varying up to 30 min, at which time the solution was removed from the vessel and filtered. The sulphur produced was washed, dried, and weighed. A small amount (3 ml) of solution was removed for analysis of nitrilotriacetic acid. The remainder of the solution was then returned to the vessel, and a small amount of $H_2SO_4$ was added to bring the pH back to 7. The general procedure was followed for 5 cycles, and the filtration, acid addition (if necessary), analysis, etc was repeated. A total of 15 cycles was run, and 30 microlitres of 1-decanol were added after the 6th and 12th cycles. The difference in weight between initial weight of nitrilotriacetic acid ligand and that remaining after 15 cycles was calculated, and is a measure of loss of ligand per unit weight of sulphur produced. The result is shown in Table III.

Example 21

A procedure similar to that of Comparative Experiment F was followed, except that the solution also contained 0.05M thiodiglycolic acid. The result is shown in Table III.

Example 22

A procedure similar to that of Comparative Experiment F was followed, except that the solution contained 0.05M 3,3-thiodipropionic acid. The result is shown in Table III.

TABLE III

| | First stabilizing composition | Grams of ligand lost per gram of sulphur produced |
|---|---|---|
| Comparative Experiment F | none | 0.14 |
| Example 21 | thiodiglycolic acid | 0.09 |
| Example 22 | 3,3-thiodipropionic acid | 0.11 |

Comparative Experiment G

An aqueous solution (150 ml) of the $Fe^{+++}$ chelate of nitrilotriacetic acid was placed in a vessel, and a stream of pure $H_2S$ was sparged into the solution with rapid stirring. Temperature of the solution was 35°C, and the solution contained 0.27 mol per litre iron as $Fe^{+++}$. Nitrilotriacetic acid ligand was present in 40 per cent mol excess, basis the iron. Sixty microlitres of 1-decanol (300 parts by million by weight) were also added to the solution. The pH of the solution was 7, and pressure was atmospheric. Addition of the $H_2S$ (360 ml) was continued until approximately 70 per cent of the $Fe^{+++}$ was converted to the $Fe^{++}$ state, which took about 2 to 3 min. The flow of the $H_2S$ and the stirring action were then discontinued, oxygen in excess was sparged into the solution, and stirring resumed for 15 min, thus regenerating the $Fe^{+++}$, and completing one cycle. The procedure was repeated for 5 cycles, the time of regeneration varying up to 30 min, at which time the solution was removed from the vessel and filtered. The sulphur produced was washed, dried, and weighed. A small amount (3 ml) of solution was removed for analysis of nitrilotriacetic acid. The remainder of the solution was then returned to the vessel, and a small amount of $H_2SO_4$ was added to bring the pH back to 7. The general procedure was followed for 5 cycles, and the filtration, acid addition (if necessary), analysis, etc. was repeated. A total of 15 cycles

was run, and 30 microlitres of 1-decanol were added after the 6th and 12th cycles. The difference in weight between initial weight of nitrilotriacetic acid ligand and that remaining after 15 cycles was calculated, and is a mesaure of loss of ligand per unit weight of sulphur produced. The result is shown in Table IV.

Example 23

A procedure similar to that of Comparative Experiment G was followed, except that the solution also contained 0.1M sodium thiocyanate. The result is shown in Table IV.

Example 24

A procedure similar to that of Comparative Experiment G was followed, except that the solution contained 0.1M sodium dithionite. The result is shown in Table IV.

TABLE IV

| | Second stabilizing composition | Grams of ligand lost per gram of sulphur produced |
|---|---|---|
| Comparative Experiment G | none | 0.14 |
| Example 23 | sodium thiocyanate | 0.06 |
| Example 24 | sodium dithionite | 0.06 |

**Claims**

1. A process for the removal of $H_2S$ from a sour gaseous stream, in which process

a) the sour gaseous stream is contacted in a contacting zone at a temperature below the melting point of sulphur with a reactant solution containing an effective amount of an oxidizing reactant comprising one or more ions of polyvalent metals and/or one or more chelate compounds of polyvalent metals, and

b) a sweet gaseous stream is separated from an admixture containing crystalline sulphur and a reduced reactant, and subsequently

c) at least a portion of the said crystalline sulphur is isolated, characterized in that an effective amount of a modifier comprising one or more alkanols having from 4 to 18 carbon atoms per molecule and/or one or more alkenols having from 4 to 22 carbon atoms per molecule is added to the reactant solution in step a) and is recovered together with the reduced reactant in the admixture of step b).

2. A process as claimed in claim 1, characterized in that the alkanol or alkanols is (are) added in step a) in an amount from 0.01 to 4% by weight, calculated on the reactant solution.

3. A process as claimed in claim 1 or 2, characterized in that the reduced reactant present in the admixture separated in step b) or left behind subsequent to step c), is contacted in a regeneration zone with an oxygen-containing gas, producing a regenerated reactant-containing admixture.

4. A process as claimed in claim 3, characterized in that the regenerated reactant-containing admixture is returned to the contacting zone in step a).

5. A process as claimed in any one of the preceding claims, characterized in that the reactant solution in step a) is an aqueous solution.

6. A process as claimed in claim 5, characterized in that the modifier is added in an amount at or near the saturation level thereof in the aqueous reactant solution.

7. A process as claimed in claim 3 or 4 and in claim 5 or 6, characterized in that step c) comprises the following substeps:—

d) separating a froth containing sulphur and aqueous admixture from the regeneration zone,

e) contacting the froth separated in substep d) with a first liquid extracting composition comprising one or more alkanols having from 4 to 15 carbon atoms per molecule and/or one or more alkenols having from 4 to 20 carbon atoms per molecule in an amount sufficient to extract more than 50 per cent of the sulphur from the froth and forming an upper liquid phase comprising solid sulphur and said first extracting composition in contact with a lower phase comprising regenerated aqueous admixture;

f) separating at least a portion of the upper liquid phase, and

g) recovering sulphur from the portion separated in substep f).

8. A process as claimed in claim 7, characterized in that at least a portion of the upper phase separated in substep f) is after sulphur recovery in substep g), returned to substep e) for contacting with froth.

9. A process as claimed in claim 7 or 8, characterized in that at least a portion of the lower phase in substep e) is separated and returned to the contacting zone in step a).

10. A process as claimed in claim 9, characterized in that the portion separated is, prior to return

to step a), contacted with an additional amount of the said first extracting composition, and at least a portion of the additional first extracting composition containing sulphur is separated.

11. A process as claimed in any one of claims 7 to 10, characterized in that the first extracting composition is used in substep e) in an amount from 50 to 300% by volume, calculated on the polyvalent metal ion solution and/or polyvalent metal chelate solution in the froth.

12. A process as claimed in claim 5 or 6, characterized in that step c) comprises the following substeps:—

k) contacting aqueous admixture separated in step b) or regenerated reactant-containing aqueous admixture with a second liquid extracting composition comprising one or more alkanols having from 4 to 15 carbon atoms per molecule,

l) forming an upper liquid phase comprising more than 50 per cent of the solid sulphur and said second extracting composition in contact with a lower phase comprising aqueous admixture containing reduced reactant or regenerated reactant, respectively,

m) separating at least a portion of the upper phase, and

n) recovering sulphur from the portion separated in substep m).

13. A process as claimed in claim 12, characterized in that at least a portion of the upper phase separated in substep m) is, after sulphur removal in substep n), returned to substep k) and contacted with aqueous admixture separated in step b) or with regenerated reactant-containing aqueous admixture.

14. A process as claimed in claim 12 or 13, characterized in that the second extracting composition is used in substep k) in an amount from 1.0 to 200% by volume, calculated on the aqueous polyvalent metal ion and/or polyvalent metal chelate solution.

15. A process as claimed in claim 14, characterized in that the second extracting composition is used in step k) in an amount from 5.0 to 120% by volume, calculated on the polyvalent metal ion and/or polyvalent metal chelate solution.

16. A process as claimed in any one of claims 7 to 10 and 12 to 15, in which the sulphur is recovered by melting from the portion separated in substeps f) and m), respectively.

17. A process as claimed in any one of claims 12 to 16, in which the aqueous admixture separated in step b) or the regenerated reactant-containing aqueous admixture is separated into an aqueous solution containing reduced reactant or regenerated reactant, respectively, and having reduced sulphur content and an aqueous reactant slurry having increased sulphur content and the said aqueous reactant slurry having increased sulphur content is contacted in substep k) with the said second extracting composition.

18. A process as claimed in claim 17, in which the said aqueous reactant slurry having increased sulphur content comprises from 2% to 30% by volume of the total of the aqueous admixture separated in step b) or of the regenerated reactant-containing aqueous admixture.

19. A process as claimed in any one of claims 1 to 4, in which the sour gaseous stream also contains $CO_2$ and a liquid absorbent selective for $CO_2$ is added to the reactant solution in step a), the admixture separated in step b) also containing absorbed $CO_2$.

20. A process as claimed in claim 19, characterized in that the liquid absorbent selective for $CO_2$ is selective for $H_2S$ as well.

21. A process as claimed in claim 19 or 20, characterized in that prior or subsequent to sulphur isolation in step c), $CO_2$ is stripped from the admixture separated in step b) and containing reduced reactant and the modifier.

22. A process as claimed in any one of the preceding claims, characterized in that one or more alkanols having from 4 to 12 carbon atoms per molecule are added as the modifier in step a).

23. A process as claimed in claim 22, characterized in that t-butanol, n-pentanol, n-octanol, n-decanol, n-undecanol and/or n-dodecanol are added as the modifier in step a).

24. A process as claimed in any one of claims 7 to 23, characterized in that one or more alkanols having from 10 to 14 carbon atoms per molecule are added to the first and second liquid extracting composition in substep e) and k), respectively.

25. A process as claimed in any one of claims 7 to 23, characterized in that one or more alkenols having from 14 to 18 carbon atoms per molecule are added to the first liquid extracting composition in step e).

26. A process as claimed in any one of claims 1 to 21, characterized in that the first and second liquid extracting composition have a melting point of not more than 60°C.

27. A process as claimed in any one of the preceding claims, characterized in that the reactant solution contains one or more polyvalent metal chelate compounds and a stabilizing amount of a first stabilizing composition comprising thiodiglycolic acid and/or 3,3-thiodipropionic acid.

28. A process as claimed in claim 27, characterized in that the first stabilizing composition is added in an amount from 0.005 to 0.3 mol per litre of solution.

29. A process as claimed in any one of the preceding claims, characterized in that the reactant solution contains one or more polyvalent metal chelate compounds and a stabilizing amount of a second stabilizing composition comprising sodium thiocyanate and/or sodium dithionite.

30. A process as claimed in claim 29, characterized in that the second stabilizing composition is added in an amount from 0.01 to 0.5 mol per litre of solution.

31. A process as claimed in any one of the preceding claims, characterized in that the reactant is a coordination complex of a polyvalent metal with an acid having the general formula:—

$$(X)_{3-n}—N—(Y)_n$$

wherein n is an integer from 1 to 3, Y represents a carboxymethyl or 2-carboxyethyl group and X a 2-hydroxyethyl or 2-hydroxypropyl group or an alkyl group having from 1 to 4 carbon atoms, or

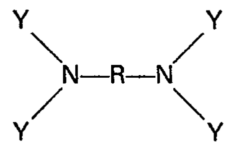

wherein:

— from 2 to 4 of the groups Y represent carboxymethyl or 2-carboxyethyl groups;

— from 0 to 2 of the groups Y represent a 2-hydroxyethyl or 2-hydroxypropyl group and

— R represents an ethylene, a trimethylene, 1-methylethylene, 1,2-cyclohexylene or 1,2-benzylene group;

or with a mixture of such acids.

32. A process as claimed in claim 31, characterized in that the reactant is a coordination complex of a polyvalent metal with an aminoacetic acid derived from ethylenediamine.

33. A process as claimed in claim 31, characterized in that the reactant is a coordination complex of a polyvalent metal with an aminoacetic acid derived from ammonia.

34. A process as claimed in any one of the preceding claims, characterized in that the polyvalent metal is iron.

35. A process as claimed in claim 34, characterized in that the reactant is a coordination complex of iron with 2-hydroxyethylethylenediaminetriacetic acid.

36. A process as claimed in claim 34, characterized in that the reactant is a coordination complex of iron with nitrilotriacetic acid.

37. A process as claimed in any one of the preceding claims, characterized in that the sour gaseous stream is a hydrocarbon stream or a stream derived from the gasification of coal.

**Patentansprüche**

1. Ein Verfahren für die Entfernung von $H_2S$ aus einem sauren, gasförmigen Strom, in welchem Verfahren

a) der saure, gasförmige Strom in einer Kontaktierungszone bei einer Temperatur unterhalb des Schmelzpunktes von Schwefel mit einer Lösung eines Reaktionsmittels in Berührung gebracht wird, welche eine wirksame Menge eines oxidierenden Reaktionsmittels enthält, das ein oder mehrere Ionen von mehrwertigen Metallen und/oder ein oder mehrere Chelatverbindungen von mehrwertigen Metallen umfaßt, und

b) ein schwefelarmer gasförmiger Strom von einer Mischung abgetrennt wird, welche kristallinen Schwefel und ein reduziertes Reaktionsmittel enthält, und anschließend

c) mindestens ein Anteil des besagten kristallinen Schwefels isoliert wird, dadurch gekennzeichnet, daß eine wirksame Menge eines Modifizierungsmittels, enthaltend ein oder mehrere Alkanole mit 4 bis 18 Kohlenstoffatomen je Molekül und/oder ein oder mehrere Alkenole mit 4 bis 22 Kohlenstoffatomen pro Molekül, zu der Reaktionslösung in Verfahrensstufe a) zugesetzt und zusammen mit dem reduzierten Reaktionsmittel in der Mischung von Verfahrensstufe b) wiedergewonnen wird.

2. Ein Verfahren wie in Anspruch 1 beansprucht, dadurch gekennzeichnet, daß das Alkanol oder die Alkanole in einer Menge von 0,01 bis 4 Gewichtsprozent, berechnet auf die Lösung des Reaktionsmittels, in Verfahrensstufe a) zugesetzt wird (werden).

3. Ein Verfahren wie in Anspruch 1 oder 2 beansprucht, dadurch gekennzeichnet, daß das reduzierte Reaktionsmittel, welches in der in Stufe b) abgetrennten Mischung oder in der nach Durchführung von Verfahrensstufe c) zurückbleibenden Mischung vorhanden ist, in einer Regenerierungszone mit einem sauerstoffhaltigen Gas in Berührung gebracht wird, wodurch eine regenerierte, das Reaktionsmittel enthaltende Mischung gebildet wird.

4. Ein Verfahren wie in Anspruch 3 beansprucht, dadurch gekennzeichnet, daß die regenerierte, das Reaktionsmittel enthaltende Mischung in die Kontaktierungszone von Verfahrensstufe a) zurückgeführt wird.

5. Ein Verfahren wie in irgendeinem der vorhergehenden Ansprüche beansprucht, dadurch gekennzeichnet, daß die in Verfahrensstufe a) verwendete Lösung des Reaktionsmittels eine wässrige Lösung ist.

6. Ein Verfahren wie in Anspruch 5 beansprucht, dadurch gekennzeichnet, daß das Modifizierungsmittel in einer Menge entsprechend oder nahe bei der Sättigungskonzentration in der wässrigen Lösung des Reaktionsmittels zugesetzt wird.

7. Ein Verfahren wie in Anspruch 3 oder 4 und in Anspruch 5 oder 6 beansprucht, dadurch gekennzeichnet, daß die Verfahrensstufe c) die folgenden Unterstufen umfaßt:

d) Abtrennen eines Schaums, der Schwefel und die wässrige Mischung enthält, aus der Regenerierungszone,

e) Kontaktieren des in Unterstufe d) abgetrennten Schaums mit einer ersten flüssigen extrahierenden Zusammensetzung, welche ein oder mehrere Alkanole mit 4 bis 15 Kohlenstoffatomen je Molekül und/oder ein oder mehrere Alkenole mit 4 bis 20 Kohlenstoffatomen je Molekül in einer Menge enthält, welche ausreicht, um mehr als 50 Prozent des Schwefels aus dem Schaum zu extrahieren, und Bildung einer oberen flüssigen Phase, welche festen Schwefel und die besagte erste extrahierende Zusammensetzung enthält und in Berührung steht mit einer unteren Phase, welche die regenerierte wässrige Mischung enthält;

f) Abtrennen mindestens eines Anteils der oberen flüssigen Phase; und

g) Wiedergewinnen von Schwefel aus dem in Unterstufe f) abgetrennten Anteil.

8. Ein Verfahren wie in Anspruch 7 beansprucht, dadurch gekennzeichnet, daß mindestens ein Anteil der in Unterstufe f) abgetrennten oberen Phase nach der Schwefelgewinnung in Unterstufe g) zu erneuten Kontaktierung mit dem Schaum in Unterstufe e) zurückgeführt wird.

9. Ein Verfahren wie in Anspruch 7 oder 8 beansprucht, dadurch gekennzeichnet, daß mindestens ein Anteil der in Unterstufe e) gebildeten unteren Phase abgetrennt und in die Kontaktierungszone von Verfahrensstufe a) zurückgeführt wird.

10. Ein Verfahren wie in Anspruch 9 beansprucht, dadurch gekennzeichnet, daß der abgetrennte Anteil vor der Zurückführung in die Verfahrensstufe a) mit einer zusätzlichen Menge der ersten extrahierenden Zusammensetzung in Berührung gebracht wird und daß mindestens ein Anteil dieser zusätzlichen schwefelhaltigen ersten extrahierend wirkenden Zusammensetzung abgetrennt wird.

11. Ein Verfahren wie in irgendeinem der Ansprüche 7 bis 10 beansprucht, dadurch gekennzeichnet, daß die erste extrahierend wirkende Zusammensetzung in Unterstufe e) in einer Menge von 50 bis 300 Volumenprozent, berechnet auf die mehrwertiges Metallion enthaltende Lösung und/oder mehrwertiges Metallchelat enthaltende Lösung in dem Schaum, angewendet wird.

12. Ein Verfahren wie in Anspruch 5 oder 6 beansprucht, dadurch gekennzeichnet, daß die Verfahrensstufe c) die folgenden Unterstufen umfaßt:

k) Kontaktieren der in Verfahrensstufe b) abgetrennten wässrigen Mischung oder der regenerierten, das Reaktionsmittel enthaltenden wässrigen Mischung mit einer zweiten flüssigen extrahierend wirkenden Zusammensetzung, welche ein oder mehrere Alkanole mit 4 bis 15 Kohlenstoffatomen je Molekül enthält,

l) Bildung einer oberen flüssigen Phase, die mehr als 50 Prozent festen Schwefel und besagte zweite extrahierend wirkende Zusammensetzung enthält und in Berührung steht mit einer unteren Phase, welche aus der das reduzierte Reaktionsmittel oder regenerierte Reaktionsmittel enthaltenden wässrigen Mischung besteht,

m) Abtrennen mindestens eines Anteils der oberen Phase und

n) Wiedergewinnung von Schwefel aus dem in Unterstufe m) abgetrennten Anteil.

13. Ein Verfahren wie in Anspruch 12 beansprucht, dadurch gekennzeichnet, daß mindestens ein Anteil der in Unterstufe m) abgetrennten oberen Phase nach der Schwefelentfernung in Unterstufe n) zur Unterstufe k) zurückgeführt und mit der in Verfahrensstufe b) abgetrennten wässrigen Mischung oder mit der regenerierten, das Reaktionsmittel enthaltenden wässrigen Mischung in Berührung gebracht wird.

14. Ein Verfahren wie in Anspruch 12 oder 13 beansprucht, dadurch gekennzeichnet, daß die zweite extrahierend wirkende Zusammensetzung in Unterstufe k) in einer Menge von 1,0 bis 200 Volumenprozent, berechnet auf die wässrige, mehrwertiges Metallion und/oder ein Chelat des mehrwertigen Metalles enthaltenden Lösung, verwendet wird.

15. Ein Verfahren wie in Anspruch 14 beansprucht, dadurch gekennzeichnet, daß die zweite extrahierende Zusammensetzung in Stufe k) in einer Menge von 5 bis 120 Volumenprozent, berechnet auf die Lösung des mehrwertigen Metallions und/oder des Chelates des mehrwertigen Metalles, verwendet wird.

16. Ein Verfahren wie in irgendeinem der Ansprüche 7 bis 10 und 12 bis 15 beansprucht, in welchem der Schwefel aus dem in den Unterstufen f) und m) abgetrennten Anteil durch Aufschmelzen wiedergewonnen wird.

17. Ein Verfahren wie in irgendeinem der Ansprüche 12 bis 16 beansprucht, in welchem die in Verfahrensstufe b) abgetrennte wässrige Mischung oder die regenerierte, das Reaktionsmittel enthaltende wässrige Mischung in eine das reduzierte Reaktionsmittel oder das regenerierte Reaktionsmittel enthaltende wässrige Lösung mit vermindertem Schwefelgehalt und eine wässrige, das Reaktionsmittel enthaltende Aufschlämmung mit erhöhtem Schwefelgehalt aufgetrennt wird und daß die besagte wässrige, das Reaktionsmittel enthaltende Aufschlämmung mit erhöhtem Schwefelgehalt in Unterstufe k) mit der besagten zweiten extrahierend wirkenden Zusammensetzung kontaktiert wird.

18. Ein Verfahren gemäß Anspruch 17, in welchem die besagte wässrige, das Reaktionsmittel enthaltende Aufschlämmung mit erhöhtem Schwefelgehalt 2 bis 30 Volumenprozent der Gesamtmenge der in Verfahrensstufe b) abgetrennten wässrigen Mischung oder der regenerierten, das Reaktionsmittel enthaltenden wässrigen Mischung ausmacht.

19. Ein Verfahren wie in irgendeinem der Ansprüche 1 bis 4 beansprucht, in welchem der saure gasförmige Strom außerdem $CO_2$ enthält und in Verfahrensstufe a) ein flüssiges, für $CO_2$ selektiv wirkendes Absorptionsmittel zu der Reaktionslösung zugesetzt wird, so daß die in Verfahrensstufe b) abgetrennte Mischung auch absorbiertes $CO_2$ enthält.

20. Ein Verfahren wie in Anspruch 19 beansprucht, dadurch gekennzeichnet, daß das für $CO_2$ selektiv wirkende flüssige Absorptionsmittel auch selektiv für $H_2S$ wirkt.

21. Ein Verfahren wie in Anspruch 19 oder 20 beansprucht, dadurch gekennzeichnet, daß vor oder im Anschluß an die Schwefelisolierung in Verfahrensstufe c) $CO_2$ aus der in Verfahrensstufe b) abgetrennten Mischung, welche reduziertes Reaktionsmittel und das Modifizierungsmittel enthält, abgestreift wird.

22. Ein Verfahren wie in irgendeinem der vorhergehenden Ansprüche beansprucht, dadurch gekennzeichnet, daß ein oder mehrere Alkanole mit 4 bis 12 Kohlenstoffatomen im Molekül als Modifizierungsmittel in Verfahrensstufe a) zugesetzt wird (werden).

23. Ein Verfahren wie in Anspruch 22 beansprucht, dadurch gekennzeichnet, daß t-Butanol, n-Pentanol, n-Octanol, n-Decanol, n-Undecanol und/oder n-Dodecanol als Modifizierungsmittel in Verfahrensstufe a) zugesetzt werden.

24. Ein Verfahren wie in irgendeinem der Ansprüche 7 bis 23 beansprucht, dadurch gekennzeichnet, daß ein oder mehrere Alkanole mit 10 bis 14 Kohlenstoffatomen je Molekül zu der ersten und zweiten flüssigen, extrahierend wirkenden Zusammensetzung in Unterstufe e) und k) zugesetzt wird (werden).

25. Ein Verfahren wie in irgendeinem der Ansprüche 7 bis 23 beansprucht, dadurch gekennzeichnet, daß ein oder mehrere Alkenole mit 14 bis 18 Kohlenstoffatomen je Molekül zu der ersten flüssigen, extrahierend wirkenden Zusammensetzung in Verfahrensstufe e) zugesetzt wird (werden).

26. Ein Verfahren wie in irgendeinem der Ansprüche 1 bis 21 beansprucht, dadurch gekennzeichnet, daß die erste und die zweite flüssige, extrahierend wirkende Zusammensetzung je einen Schmelzpunkt von nicht mehr als 60°C aufweist.

27. Ein Verfahren wie in irgendeinem der vorhergehenden Ansprüche beansprucht, dadurch gekennzeichnet, daß die das Reaktionsmittel enthaltende Lösung ein oder mehrere Chelatverbindungen eines mehrwertigen Metalles und eine stabilisierend wirkende Menge einer ersten stabilisierend wirkenden Zusammensetzung enthält, welche Thiodiglykolsäure und/oder 3,3-Thiodipropionsäure enthält.

28. Ein Verfahren wie in Anspruch 27 beansprucht, dadurch gekennzeichnet, daß die erste stabilisierend wirkende Zusammensetzung in einer Menge von 0,005 bis 0,3 Mol je Liter Lösung zugesetzt wird.

29. Ein Verfahren wie in irgendeinem der vorhergehenden Ansprüche beansprucht, dadurch gekennzeichnet, daß die das Reaktionsmittel enthaltende Lösung ein oder mehree Chelatverbindungen eines mehrwertigen Metalls und eine stabilisierende Menge einer zweiten stabilisierend wirkenden Zusammensetzung enthält, welche Natriumthiocyanat und/oder Natriumdithionit enthält.

30. Ein Verfahren wie in Anspruch 29 beansprucht, dadurch gekennzeichnet, daß die zweite stabilisierend wirkende Zusammensetzung in einer Menge von 0,01 bis 0,5 Mol je Liter Lösung zugesetzt wird.

31. Ein Verfahren wie in irgendeinem der vorhergehenden Ansprüche beansprucht, dadurch gekennzeichnet, daß das Reaktionsmittel ein Koordinationskomplex eines mehrwertigen Metalles mit einer Säure der nachstehenden Formel ist:

$$(X)_{3-n}—N—(Y)_n$$

in welcher n eine ganze Zahl von 1 bis 3 ist, Y eine Carboxymethyl- oder 2-Carboxyäthylgruppe bedeutet und X eine 2-Hydroxyäthyl- oder 2-Hydroxypropylgruppe oder eine Alkylgruppe mit 1 bis 4 Kohlenstoffatomen ist, oder einer Säure der nachstehenden Formel:

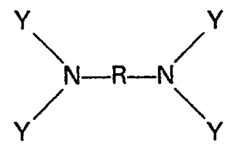

in welcher

2 bis 4 der Gruppen Y Carboxymethyl- oder 2-Carboxyäthylgruppen sind;

0 bis 2 der Gruppen Y eine 2-Hydroxyäthyl- oder 2-Hydroxypropylgruppe darstellen und

R eine Athylen-, Trimethylen-, 1-Methyläthylen-, 1,2-Cyclohexylen- oder 1,2-Benzylengruppe bedeutet oder ein Koordinationskomplex einer Mischung solcher Säuren ist.

32. Ein Verfahren wie in Anspruch 31 beansprucht, dadurch gekennzeichnet, daß das Reaktionsmittel ein Koordinationskomplex eines mehrwertigen Metalles mit einer von Äthylendiamin abgeleiteten Aminoessigsäure ist.

33. Ein Verfahren wie in Anspruch 31 beansprucht, dadurch gekennzeichnet, daß das Reaktionsmittel ein Koordinationskomplex eines mehrwertigen Metalles einer von Ammoniak abgeleiteten Aminoessigsäure ist.

34. Ein Verfahren wie in einem der vorhergehenden Ansprüche beansprucht, dadurch gekennzeichnet, daß das mehrwertige Metall Eisen ist.

35. Ein Verfahren wie in Anspruch 34 beansprucht, dadurch gekennzeichnet, daß das Reaktionsmittel ein Koordinationskomplex von Eisen mit 2-Hydroxyäthylendiamintriessigsäure ist.

36. Ein Verfahren wie in Anspruch 34 beansprucht, dadurch gekennzeichnet, daß das Reaktionsmittel ein Koordinationskomplex von Eisen mit Nitrilotriessigsäure ist.

37. Ein Verfahren wie in irgendeinem der vorhergehenden Ansprüche beansprucht, dadurch gekennzeichnet, daß der saure gasförmige Strom ein Kohlenwasserstoffstrom oder ein bei der Vergasung von Kohle entstehender Strom ist.

**Revendications**

1. Procédé d'enlèvement d'$H_2S$ d'un courant gazeux corrosif, où

a) on met en contact le courant gazeux corrosif dans une zone de contact à une température inférieure au point de fusion du soufre avec une solution réactive contenant une quantité efficace d'un réactif oxydant comprenant un ou plusieurs ions de métaux polyvalents et/ou un ou plusieurs composés chélatés de métaux polyvalents, et

b) on sépare un courant gazeux non corrosif d'un mélange contenant du soufre cristallin et un réactif réduit, puis

c) on isole au moins une fraction dudit soufre cristallin, caractérisé en ce qu'on ajoute à la solution réactive dans l'étape a) une quantité efficace d'un modificateur comprenant un ou plusieurs alcanols ayant de 4 à 18 atomes de carbone par molécule et/ou un ou plusieurs alcénols ayant de 4 à 22 atomes de carbone par molécule et en ce qu'on la recueille avec le réactif réduit dans le mélange de l'étape b).

2. Procédé selon la revendication 1, caractérisé en ce qu'on ajoute le ou les alcanols dans l'étape a) en une quantité allant de 0,01 à 4% en poids, calculée par rapport à la solution réactive.

3. Procédé selon les revendications 1 et 2, caractérisé en ce qu'on met en contact le réactif réduit présent dans le mélange séparé dans l'étape b) ou laissé après l'étape c) dans une zone de régénération avec un gaz contenant de l'oxygène, produisant un mélange régénéré contenant le réactif.

4. Procédé selon la revendication 3, caractérisé en ce qu'on renvoie le mélange régénéré contenant le réactif vers la zone de contact de l'étape a).

5. Procédé selon l'une quelconque des revendications précédentes, caractérisé en ce que la solution réactive dans l'étape a) est une solution aqueuse.

6. Procédé selon la revendication 5, caractérisé en ce que le modificateur est ajouté en une quantité qui est ou avoisine son niveau de saturation dans la solution reactive aqueuse.

7. Procédé selon les revendications 3 ou 4 et les revendications 5 ou 6, caractérisé en ce que l'étape c) comprend les sous-étapes suivantes:

d) séparation d'une mousse contenant du soufre et du mélange aqueux à partir de la zone de régénération,

e) mise en contact de la mousse séparée dans la sous-étape d) avec une première composition d'extraction de liquide comprenant un ou plusieurs alcanols ayant de 4 à 15 atomes de carbone par molécule et/ou un ou plusieurs alcénols ayant de 4 à 20 atomes de carbone par molécule en une quantité suffisante pour extraire plus de 50% du soufre à partir de la mousse et formation d'une phase liquide supérieure comprenant du soufre solide et ladite première composition d'extraction en contact avec une phase inférieure comprenant un mélange aqueux régénéré;

f) séparation d'au moins une fraction de la phase liquide supérieure, et

g) récupération du soufre à partir de la fraction séparée dans la sous-étape f).

8. Procédé selon la revendication 7, caractérisé en ce qu'on renvoie au moins une fraction de la phase supérieure séparée dans la sous-étape f), après récupération du soufre dans la sous-étape g), vers la sous-étape e) pour la mettre en contact avec la mousse.

9. Procédé selon les revendications 7 et 8, caractérisé en ce qu'on sépare au moins une fraction de la phase inférieure dans la sous-étape e) et en ce qu'on la renvoie vers la zone de contact de l'étape a).

10. Procédé selon la revendication 9, caractérisé en ce qu'on met en contact la fraction séparée, avant de la renvoyer à l'étape a), avec une quantité supplémentaire de ladite première composition d'extraction, et en ce qu'on sépare au moins une fraction de la première composition d'extraction additionnelle contenant du soufre.

11. Procédé selon l'une quelconque des revendications 7 à 10, caractérisé en ce que la première

composition d'extraction est utilisée dans la sous-étape e) en une quantité allant de 50 à 300% en volume, calculée par rapport à la solution d'ion métallique polyvalent et/ou de chélate métallique polyvalent dans la mousse.

12. Procédé selon les revendications 5 et 6, caractérisé en ce que l'étape c) comprend les sous-étapes suivantes:

k) mise en contact du mélange aqueux séparé dans l'étape b) ou du mélange aqueux régénéré contenant le réactif avec une seconde composition d'extraction liquide comprenant un ou plusieurs alcanols ayant de 4 à 15 atomes de carbone par molécule,

l) formation d'une phase liquide supérieure comprenant plus de 50% du soufre solide et ladite seconde composition d'extraction en contact avec une phase inférieure comprenant un mélange aqueux contenant le réactif réduit ou le réactif régénéré, respectivement,

m) separation d'au moins une fraction de la phase supérieure, et

n) récupération du soufre à partir de la fraction séparée dans la sous-étape m).

13. Procédé selon la revendication 12, caractérisé en ce qu'au moins une fraction de la phase supérieure séparée dans la sous-étape m) est, après enlèvement du soufre dans la sous-etape n) renvoyée vers la sous-étape k) et mise en contact avec le mélange aqueux séparée dans l'étape b) ou avec le mélange aqueux régénéré contenant le réactif.

14. Procédé selon les revendications 12 et 13, caractérisé en ce que la seconde composition d'extraction est utilisée dans la sous-étape k) en une quantité allant de 1,0 à 200% en volume, calculée par rapport à la solution aqueuse d'ion métallique polyvalent et/ou de chélate métallique polyvalent.

15. Procédé selon la revendication 14, caractérisé en ce que la seconde composition d'extraction est utilisée dans l'étape k) en une quantité allant de 5,0 à 120% en volume, calculée sur la base de la solution d'ion metallique polyvalent et/ou de chelate métallique polyvalent.

16. Procédé selon l'une quelcone des revendications 7 à 10 et 12 à 15, où l'on recueille le soufre en le faisant fondre à partir de la fraction séparée dans les sous-étapes f) et m), respectivement.

17. Procédé selon l'une quelconque des revendications 12 à 16, où l'on sépare le mélange aqueux séparé dans l'étape b) ou le mélange aqueux contenant le réactif régénéré en une solution aqueuse contenant le réactif réduit ou le réactif régénéré, respectivement, et ayant une teneur en soufre réduite et une bouillie réactive aqueuse ayant une teneur en soufre accrue et l'on met en contact ladite bouillie réactive aqueuse ayant une teneur en soufre accrue dans la sous-étape k) avec ladite seconde composition d'extraction.

18. Procédé selon la revendication 17, où ladite bouillie réactive aqueuse ayant une teneur en soufre accrue comprend de 20 à 30% en volume du total du mélange aqueux séparé dans l'étape b) ou du mélange aqueux contenant le réactif régénéré.

19. Procédé selon l'une quelconque des revendications 1 à 4, où le courant gazeux corrosif contient également du $CO_2$ et ou l'on ajoute un absorbant liquide selectif pour $CO_2$ à la solution réactive de l'étape a), le melange sépare dans l'étape b) contenant également du $CO_2$ absorbé.

20. Procédé selon la revendication 19, caractérisé en ce que l'absorbant liquide sélectif pour $CO_2$ est également sélectif pour $H_2S$.

21. Procédé selon les revendications 19 et 20, caractérisé en ce qu'avant ou après l'isolement du soufre dans l'étape c), on enlève le $CO_2$ du mélange séparé dans l'étape b) et contenant le réactif réduit et le modificateur.

22. Procédé selon l'une quelconque des revendications précédentes, caractérisé en ce qu'on ajoute un ou plusieurs alcanols ayant de 4 à 12 atomes de carbone par molécule comme modificateur dans l'étape a).

23. Procédé selon la revendication 22, caractérisé en ce qu'on ajoute du t-butanol, du n-pentanol, du n-octanol, du n-décanol, du n-undécanol et/ou du n-dodécanol comme modificateur dans l'étape a).

24. Procédé selon l'une quelconque des revendications 7 à 23, caractérisé en ce qu'on ajoute un ou plusieurs alcanols ayant de 10 à 14 atomes de carbone par molécule à la première et à la seconde compositions d'extraction de liquide dans les sous-étapes e) et k), respectivement.

25. Procédé selon l'une quelconque des revendications 7 à 23, caractérisé en ce qu'on ajoute un ou plusieurs alcénols ayant de 14 à 18 atomes de carbone par molécule à la première composition d'extraction de liquide de l'étape e).

26. Procédé selon l'une quelconque des revendications 1 à 21, caractérisé en ce que les première et seconde compositions d'extraction de liquide ont un point de fusion ne dépassant pas 60°C.

27. Procédé selon l'une quelconque des revendications précédentes, caractérisé en ce que la solution réactive contient un ou plusieurs composés chélatés métalliques polyvalents et une quantité stabilisante d'une première composition de stabilisation comprenant de l'acide thiodiglycolique et/ou de l'acide 3,3-thiodipropionique.

28. Procédé selon la revendication 27, caractérisé en ce qu'on ajoute la première composition de stabilisation en une quantité allant de 0,005 à 0,3 moles par litre de solution.

29. Procédé selon l'une quelconque des revendications précédentes, caractérisé en ce que la solution réactive contient un ou plusieurs composés chelatés métalliques polyvalents et une quantité stabilisante d'une seconde composition de stabilisation comprenant du thiocyanate de sodium et/ou du dithionite de sodium.

30. Procédé selon la revendication 29, caractérisé en ce qu'on ajoute la seconde composition de stabilisation en une quantité allant de 0,01 à 0,5 moles par litre de solution.

31. Procédé selon l'une quelconque des revendications précédentes, caractérisé en ce que le réactif est une complexe de coordination d'un métal polyvalent avec un acide de formule générale

$$(X)_{3-n}—N—(Y)_n$$

où n est un nombre entier allant de 1 à 3, Y représenté un groupe carboxyméthyle ou 2-carboxyéthyle et X est un groupe 2-hydroxyéthyle ou 2-hydroxypropyle ou un groupe alcoyle en $C_1$ a $C_4$, ou

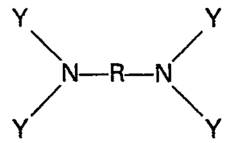

où

— de 2 à 4 des groupes Y représentent des groupes carboxyméthyle ou 2-carboxyéthyle;
— de 0 à 2 des groupes Y représentent un groupe 2-hydroxyéthyle ou 2-hydroxypropyle et
— R représente un groupe éthylène, triméthylène, 1-méthyléthylène, 1,2-cyclohexylène ou 1,2-benzylene;

ou avec un mélange de tels acides.

32. Procédé selon la revendication 31, caractérisé en ce que le réactif est un complexe de coordination d'un metal polyvalent avec un acide aminoacétique dérivé de l'éthylènediamine.

33. Procédé selon la revendication 31, caractérisé en ce que le réactif est un complexe de coordination d'un métal polyvalent avec un acide aminoacétique dérivé de l'ammoniac.

34. Procédé selon l'une quelconque des revendications precedentes, caractérisé en ce que le métal polyvalent est le fer.

35. Procédé selon la revendication 34, caractérisé en ce que le réactif est un complexe de coordination de fer avec l'acide 2-hydroxyéthyléthylènediaminetriacétique.

36. Procédé selon la revendication 34, caractérisé en ce que le réactif est un complexe de coordination de fer avec l'acide nitrilotriacetique.

37. Procédé selon l'une quelconque des revendications précédentes, caractérisé en ce que le courant gazeux corrosif est un courant hydrocarboné ou un courant dérivé de la gazéification du charbon.

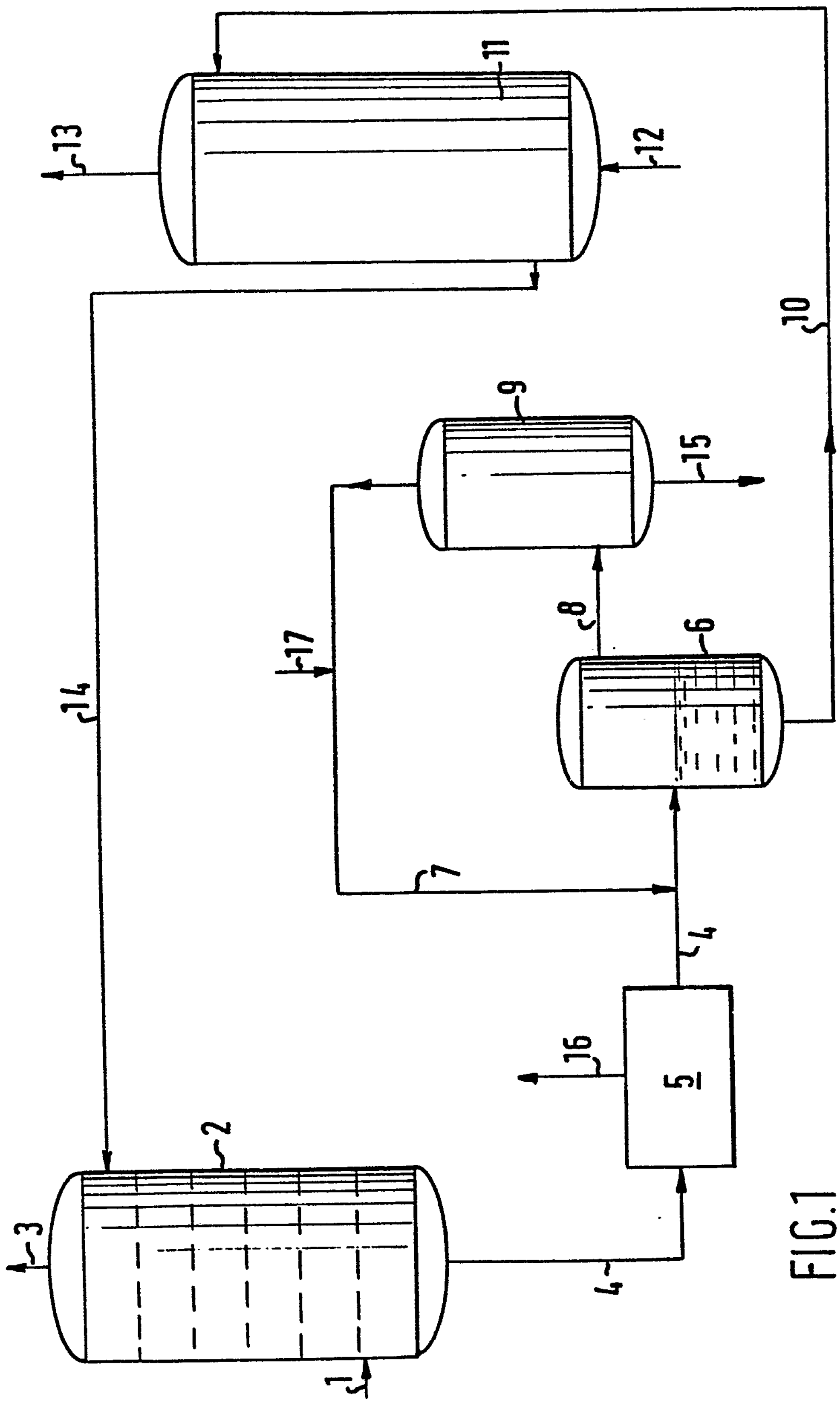


FIG.1

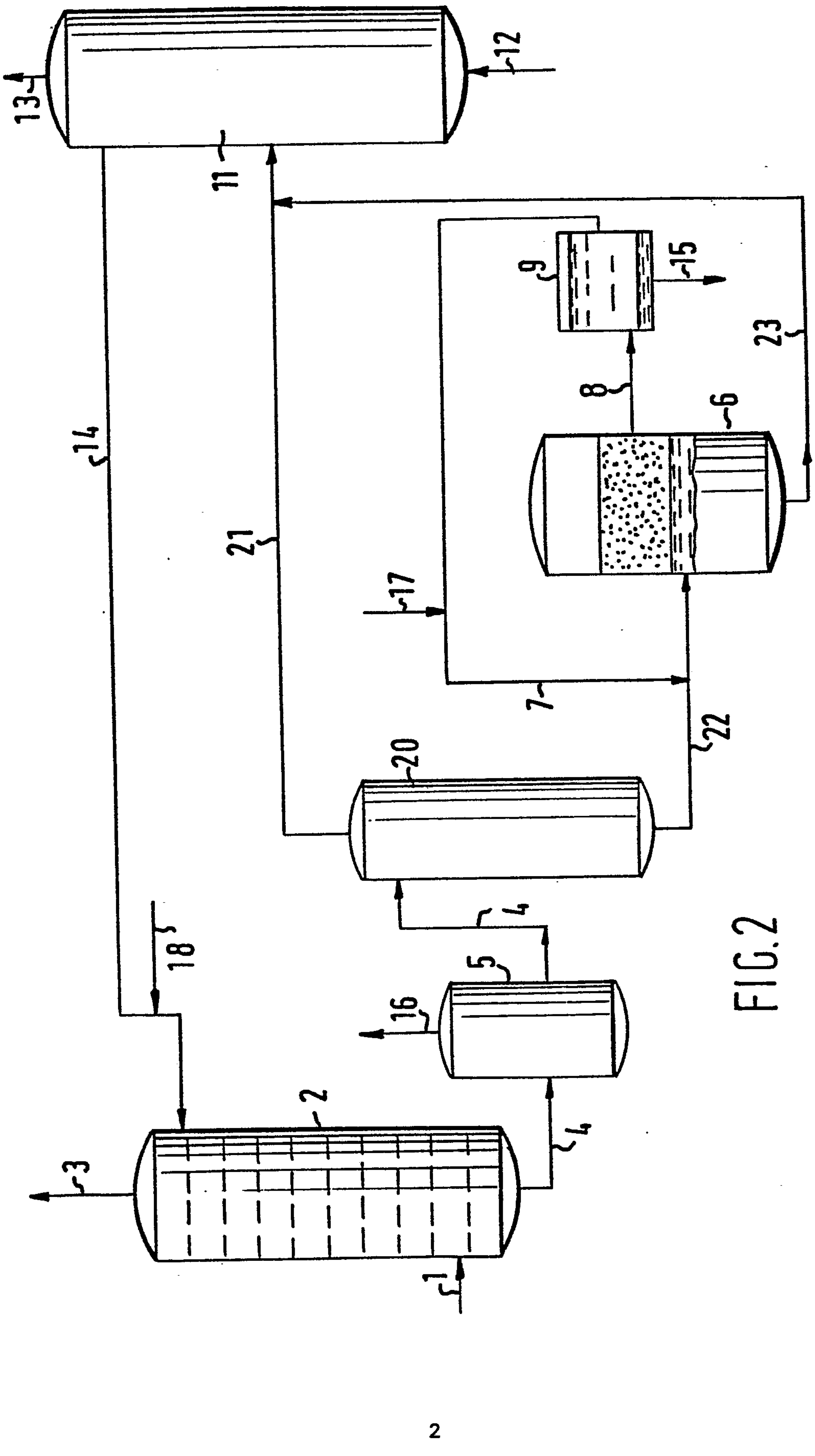


FIG.2

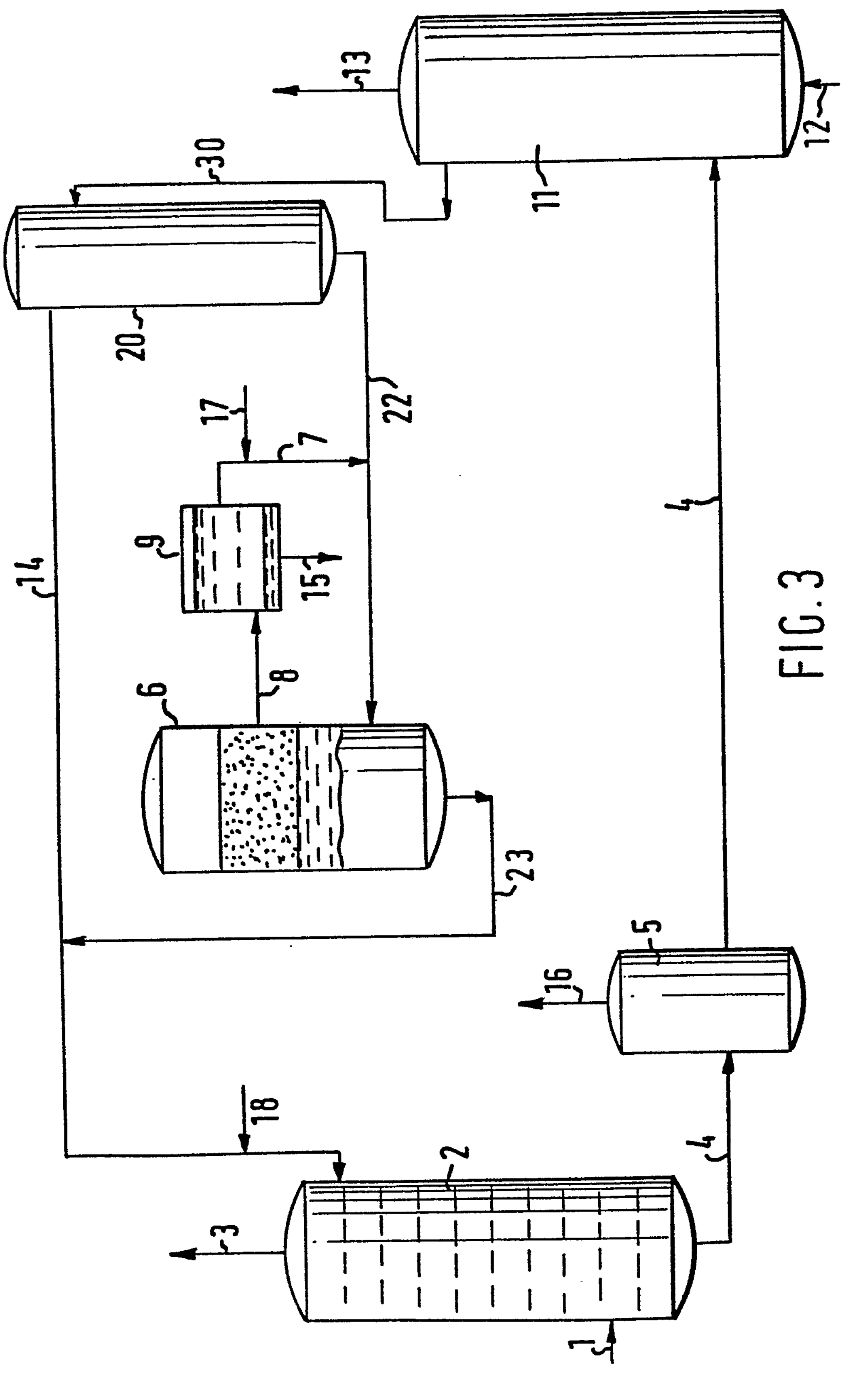


FIG. 3

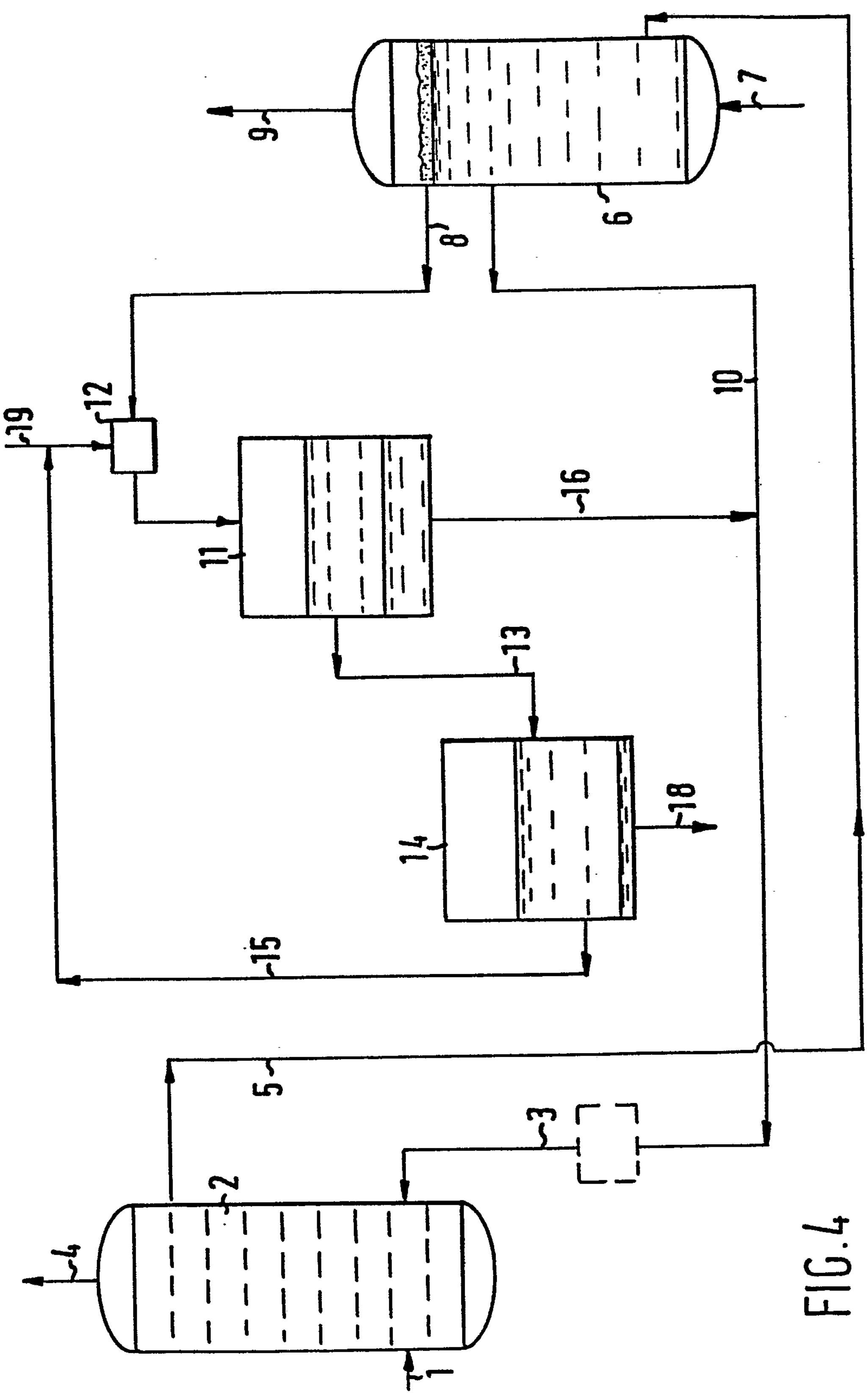


FIG.4

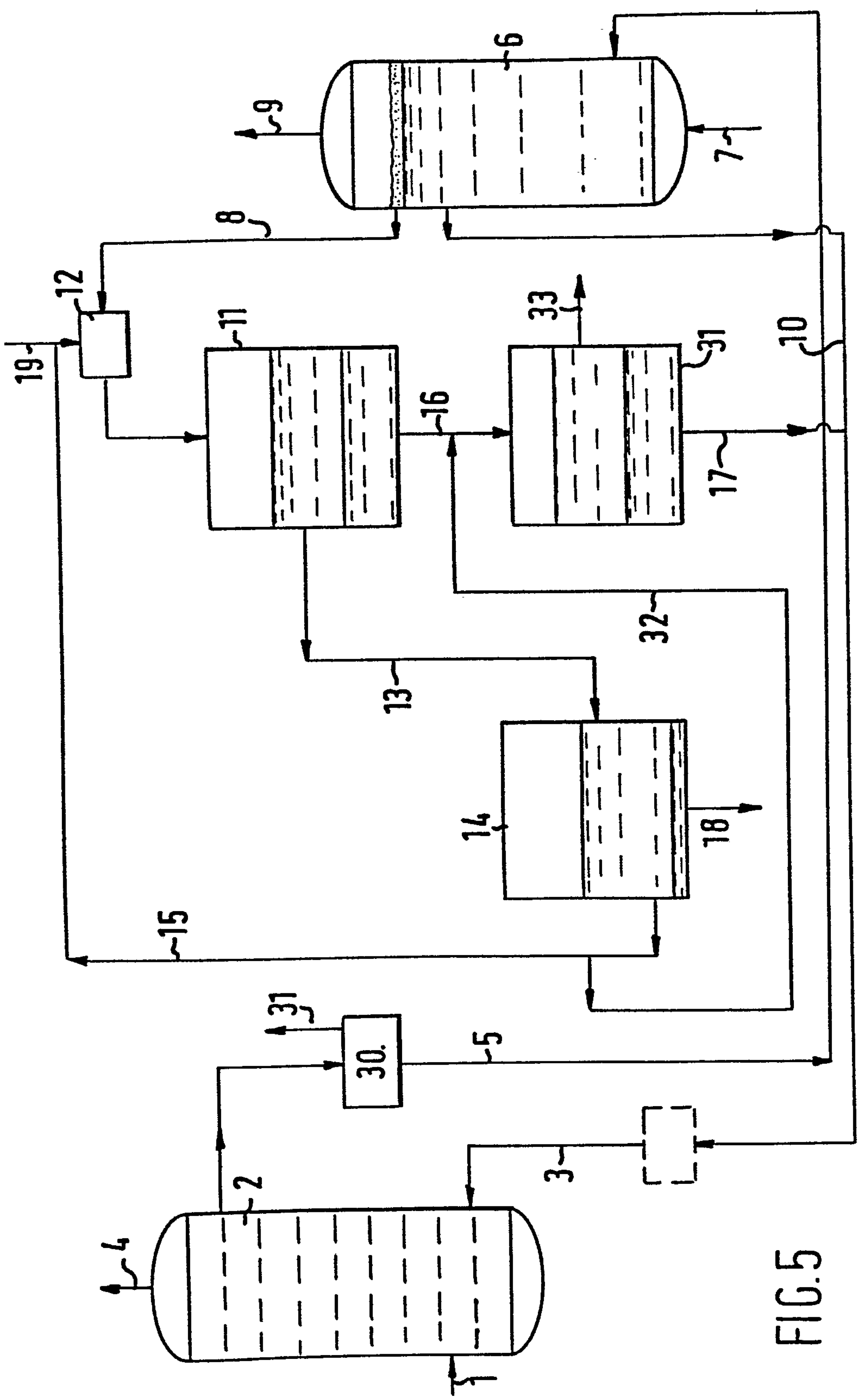


FIG.5